# EUROPEAN PATENT SPECIFICATION

(11) **EP 1 826 206 B1**
(45) Date of publication and mention of the grant of the patent: **08.07.2009**
(21) Application number: 07002074.8
(22) Date of filing: 31.01.2007
(51) Int. Cl.: C07D 333/76, B41N 1/00, C09D 11/00, G03F 7/00

(54) **Sulfonium salt, curable composition, ink composition, inkjet recording method, printed material, process for producing lithographic printing plate, and lithographic printing plate**
Sulfonium-Saltz, härtbare Zusammensetzung, Tintenzusammensetzung, Tintenstrahldruckverfahren, bedrucktes Material, Verfahren zur Herstellung einer lithographischen Druckplatte und lithographische Druckplatte
Sel de sulfonium, composition durcissable, composition pour encres, méthode d'impression à jet d'encre, imprimé, procédé de fabrication de planche d'impression lithographique et planche d'impression lithographique

(30) Priority: 23.02.2006 JP 2006047032; 25.07.2006 JP 2006201406
(43) Date of publication of application: 29.08.2007
(73) Proprietor: Fujifilm Corporation, Minato-ku Tokyo (JP)
(72) Inventor: Tsuchimura, Tomotaka, Haigara-gun Shizuoka (JP); Kawanishi, Yasutomo, Haigara-gun Shizuoka (JP)
(74) Representative: HOFFMANN EITLE

(56) References cited:
- WO-A-03/008404
- GB-A- 1 516 511

## Description

The present invention relates to a novel sulfonium salt, a curable composition employing same, an ink composition suitably used for inkjet recording, an inkjet recording method, a printed material employing same, a lithographic printing plate obtained using the ink composition, and a process for producing a lithographic printing plate. More particularly, it relates to a curable composition that cures with very high sensitivity and without coloration upon exposure to radiation, can form a high quality image with good color reproduction, and has good storage stability; an ink composition suitable for inkjet recording that does not cause head clogging; an inkjet recording method; a printed material employing same; a lithographic printing plate obtained using the ink composition; and a process for producing a lithographic printing plate.

With regard to an image recording method for forming an image on a recording medium such as paper based on an image data signal, there are an electrophotographic system, sublimation type and melt type thermal transfer systems, an inkjet system, etc. In the electrophotographic system, a process of forming an electrostatic latent image on a photosensitive drum by electrically charging and exposing is required, and the system is complicated; as a result, there is the problem that the production cost is high. With regard to the thermal transfer system, although the equipment is inexpensive, due to the use of an ink ribbon there is the problem that the running cost is high and waste material is generated. On the other hand, with regard to the inkjet system, the equipment is inexpensive and, since an image is formed directly on a recording medium by discharging an ink only on a required image area, the ink can be used efficiently and the running cost is low. Furthermore, there is little noise and it is excellent as an image recording system.

An ink composition that can be cured by exposure to radiation such as ultraviolet rays and, in particular, an inkjet recording ink (radiation curing type inkjet recording ink) are required to have sufficiently high sensitivity and provide a high image quality. By achieving higher sensitivity, a large number of benefits are provided, such as high curability toward radiation, a reduction in power consumption, longer lifetime due to a decrease in the load on a radiation generator, and prevention of formation of low molecular weight material originating from insufficient curing. Furthermore, higher sensitivity particularly improves the cure strength of an image formed using the ink composition and, in particular, the inkjet recording ink, particularly for the formation of a lithographic printing plate, and high plate life can be obtained.

The main function of a photoreaction initiator is to initiate a reaction by receiving radiation such as ultraviolet rays (UV). There are two main types of photoreaction initiator for initiating a reaction of a monomer or a prepolymer, that is, a radical photoreaction initiator and a cationic photoreaction initiator.

The most commonly used cationic photoreaction initiator is an organoiodonium salt or a sulfonium salt. The mechanism of action thereof is as follows: the cationic photoreaction initiator forms an excited state when it receives radiation, the excited state breaks down to thus emit a radical cation, and this radical cation reacts with a solvent or another hydrogen atom donor to finally form a protonic acid. This protonic acid is an active chemical species that initiates a reaction.

In this technical field, there is still a desire for a cation reaction initiator, such as a novel sulfonium salt, that can advantageously be used with a UV-curing composition.

On the other hand, there has been reported, for example, a sulfonium salt that absorbs light efficiently and has improved sensitivity by increasing the wavelength (ref. JP-A-2000-186071, JP-A-2005-187799, and JP-A-2005-263796; JP-A denotes a Japanese unexamined patent publication application).

However, a curable composition and an ink composition that can be cured by irradiation with radiation such as ultraviolet rays and, in particular, an inkjet recording ink composition (radiation-curing inkjet recording ink composition), are required to have sufficiently high sensitivity and provide high image quality, and when the ink composition and, in particular, the inkjet recording ink, are used as an image area of a lithographic printing plate, the image area is required to have high curing strength, that is, long plate life. Furthermore, when they are used as a white inkjet ink, there is the problem of coloration, and there is a desire for a cationically polymerizable compound that has high polymerization efficiency and that as an ink composition has excellent sensitivity to radiation and causes no coloration. In addition, since a sulfonium salt having a highly polar functional group such as a carbonyl group, a hydroxyl group, or an amino group influences the dispersion stability of a pigment and degrades the ink stability over time or the ink discharge stability, improvement is desired.

It is an object of the present invention, which has been accomplished under the above-mentioned circumstances, to provide a curable composition that can cure with high sensitivity upon exposure to actinic radiation. It is also an object thereof to provide a sulfonium salt that can suitably be used in the curable composition. It is yet another object thereof to provide an ink composition that cures with very high sensitivity and without coloration upon exposure to actinic radiation, can form a high quality image, has excellent adhesion to a recording medium, and has good storage stability, an ink composition that is suitable for inkjet recording and that does not cause head clogging, and an inkjet recording method employing the ink composition.

It is yet another object of the present invention to provide a printed material and a lithographic printing plate obtained using an ink composition that has excellent storage stability and can be cured with high sensitivity upon exposure to ultraviolet rays, and a process for producing a lithographic printing plate using the ink composition.

The above-mentioned objects have been accomplished by (1) to (5), (8), and (10) to (12). They are described below together with (6), (7), and (9), which are preferred embodiments.
(1) A sulfonium salt having a cation represented by Formula (II) 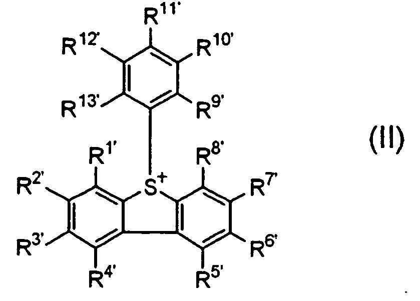 (R^{1'} to R^{13'} in Formula (II) independently denote a hydrogen atom or a substituent, and may be bonded to each other to form a ring, provided that at least one of R^{1'} to R^{8'} denotes a halogen atom or a haloalkyl group),
(2) a sulfonium salt having a cation represented by Formula (II), 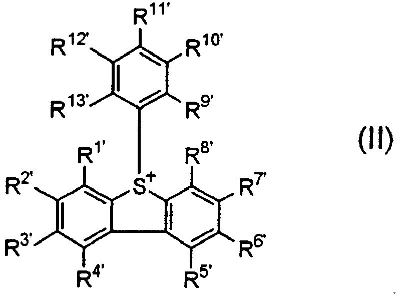 (R^{1'} to R^{13'} in Formula (II) independently denote a hydrogen atom or a substituent, and may be bonded to each other to form a ring, provided that at least two of R^{1'} to R^{8'} denote a halogen atom or a haloalkyl group),
(3) a curable composition comprising the sulfonium salt according to (1) or (2),
(4) a curable composition comprising (a) the sulfonium salt according to (1) or (2), (b) a polymerizable compound, and (c) a sensitizer,
(5) an ink composition comprising the curable composition according to (3) or (4),
(6) the ink composition according to (5), wherein it comprises (d) a colorant,
(7) the ink composition according to (5) or (6), wherein it is for inkjet recording use,
(8) an inkjet recording method comprising a step (a¹) of discharging an ink composition onto a recording medium, and a step (b¹) of irradiating the discharged ink composition with actinic radiation so as to cure the ink composition, the ink composition being the ink composition according to any one of (5) to (7),
(9) the inkjet recording method according to (8), wherein the actinic radiation is ultraviolet radiation emitted by a light emitting diode that has a light emission peak wavelength in the range of 350 to 420 nm and generates ultraviolet radiation whose maximum illumination intensity on the surface of a recording medium is 10 to 2,000 mW/cm²,
(10) a printed material recorded by the inkjet recording method according to (8) or (9),
(11) a process for producing a lithographic printing plate, the process comprising a step (a²) of discharging the ink composition according to any one of (5) to (7) onto a hydrophilic support and a step (b²) of irradiating the discharged ink composition with actinic radiation so as to cure the ink composition, thus forming a hydrophobic image on the hydrophilic support by curing the ink composition, and
(12) a lithographic printing plate produced by the process for producing a lithographic printing plate according to (11).

Since the sulfonium salt of the present invention has high planarity and has an electron-withdrawing halogen atom bonded to a benzothiophene skeleton, it has high decomposition efficiency by virtue of electron transfer and functions as an excellent photo-acid generator and a radical generator. A curable composition comprising the sulfonium salt cures with high sensitivity to radiation and gives a cured material having excellent physical properties. In accordance with the present invention, curing can be carried out with high sensitivity and without coloration, and a high quality image with good color reproduction can be formed. A sulfonium salt having a halogen atom incorporated thereinto has less influence on the dispersion stability of a pigment than a sulfonium salt having another functional group incorporated thereinto, and can provide an ink composition that has good storage stability and excellent discharge stability together with an inkjet recording method that employs the ink composition.

Furthermore, a printed material obtained using the ink composition having excellent storage stability and capable of curing with high sensitivity when exposed to ultraviolet rays has high image quality, good color reproduction, and excellent strength for an image area. Similarly, in accordance with use of the ink composition of the present invention, there is exhibited the effect that a lithographic printing plate having high plate life and high image quality can be produced based on digital data.

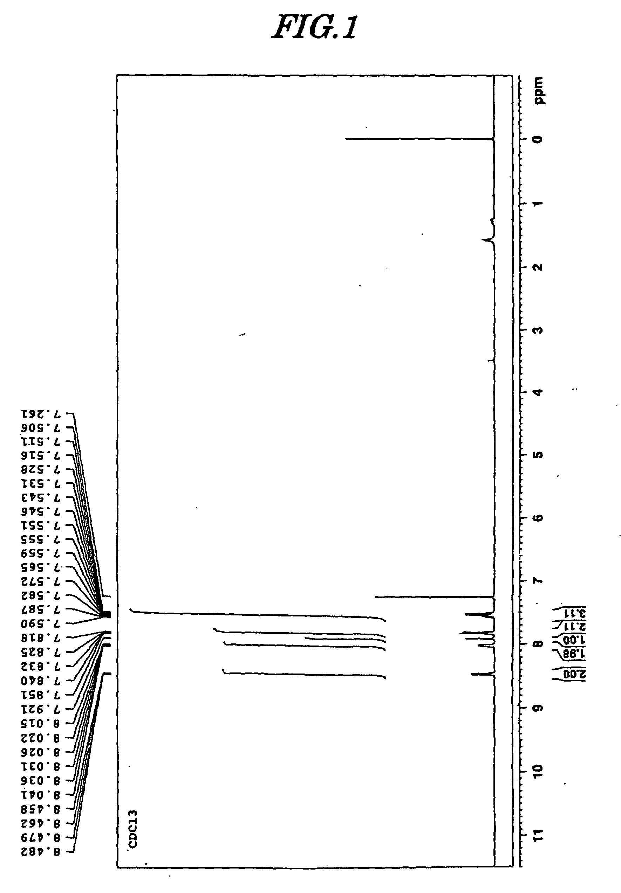

FIG. 1 is a ¹H-NMR chart of Sulfonium salt B of the present invention.

The sulfonium salt of the present invention has a cation represented by Formula (II). The sulfonium salt having a cation represented by Formula (II) of the present invention is suitably used in a curable composition.

The curable composition of the present invention comprises (a) a sulfonium salt having a cation represented by Formula (II) (hereinafter, also called 'sulfonium salt (A)'), (b) a polymerizable compound, and (c) a sensitizer.

Furthermore, the curable composition of the present invention is suitably used in an ink composition (hereinafter, also called simply an 'ink'). Moreover, the curable composition and the ink composition of the present invention may comprise as necessary (d) a colorant, (e) a cosensitizer, (f) another polymerization initiator, and (g) another component.

### (a) Sulfonium salt having cation represented by Formula (II) (sulfonium salt (A))

The sulfonium salt having a cation represented by Formula (II) (sulfonium salt (A)) of the present invention is explained in detail.

In the present invention, (a) the sulfonium salt (A) has the functions of generating a polymerization-active species when irradiated with radiation, and initiating and making proceed the polymerization of (b) a polymerizable compound. In particular, in the present invention, the sulfonium salt (A) has the functions of generating a cationic polymerization-active species, and initiating and making proceed the polymerization of a cationically polymerizable compound. Furthermore, since it generates a radical polymerization-active species, it also has the functions of initiating and making proceed the polymerization of a radically polymerizable compound.

Furthermore, when (a) the sulfonium salt (A) is used in a curable composition or an ink composition, the sulfonium salt (A) may be used singly or in a combination of two or more types.

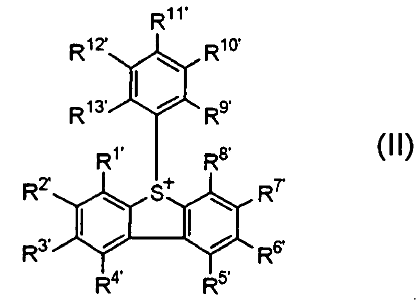

R^{1'} to R^{13'} in Formula (II) independently denote a hydrogen atom or a substituent, and may be bonded to each other to form a ring, provided that at least one of R^{1'} to R^{8'} denotes a halogen atom or a haloalkyl group.

The sulfonium salt having a cation represented by Formula (II) (sulfonium salt (A)) has a counteranion and is electrically neutral due to the counteranion.

In the present invention, when a specific portion is referred to as a 'group', it means that it may be unsubstituted or substituted with at least one type (up to a possible maximum number) of substituent unless otherwise specified. For example, an 'alkyl group' means a substituted or unsubstituted alkyl group.

Furthermore, in the present invention, when a specific portion is referred to as a 'ring' or when a 'group' contains a 'ring', unless otherwise specified it may be a monocyclic or condensed ring, and may be substituted or unsubstituted. For example, an 'aryl group' may be a phenyl group, a naphthyl group, or a substituted phenyl group.

R^{1'} to R^{13'} independently denote a hydrogen atom or a substituent, which may be any kind of substituent and is not particularly limited, and examples thereof include an alkyl group (including a cycloalkyl group, a bicycloalkyl group, and a tricycloalkyl group), an alkenyl group (including a cycloalkenyl group and a bicycloalkenyl group), an alkynyl group, an aryl group, a heterocyclic group, a cyano group, a hydroxyl group, a nitro group, a carboxyl group, an alkoxy group, an aryloxy group, a silyloxy group, a heterocyclic oxy group, an acyloxy group, a carbamoyloxy group, an alkoxycarbonyloxy group, an aryloxycarbonyloxy group, an amino group (including an anilino group), an ammonio group, an acylamino group, an aminocarbonylamino group, an alkoxycarbonylamino group, an aryloxycarbonylamino group, a sulfamoylamino group, an alkyl or aryl sulfonylamino group, a mercapto group, an alkylthio group, an arylthio group, a heterocyclothio group, a sulfamoyl group, a sulfo group, an alkyl or aryl sulfinyl group, an alkyl or aryl sulfonyl group, an aryloxycarbonyl group, an alkoxycarbonyl group, a carbamoyl group, an aryl or heterocycloazo group, an imide group, a phosphino group, a phosphinyl group, a phosphinyloxy group, a phosphinylamino group, a phosphono group, a silyl group, a hydrazino group, a ureido group, a boronic acid group (-B(OH)₂), a phosphate group (-OPO(OH)₂), a sulfate group (-OSO₃H), or another known substituent.

Moreover, two of R^{1'} to R^{13'} may together form a ring (an aromatic or nonaromatic hydrocarbon ring, or a heterocycle, which may further be combined to form a polycondensed ring; examples thereof include a benzene ring, a naphthalene ring, an anthracene ring, a phenanthrene ring, a fluorene ring, a triphenylene ring, a naphthacene ring, a biphenyl ring, a pyrrole ring, a furan ring, a thiophene ring, an imidazole ring, an oxazole ring, a thiazole ring, a pyridine ring, a pyrazine ring, a pyrimidine ring, a pyridazine ring, an indolizine ring, an indole ring, a benzofuran ring, a benzothiophene ring, an isobenzofuran ring, a quinolizine ring, a quinoline ring, a phthalazine ring, a naphthylidine ring, a quinoxaline ring, a quinazoline ring, an isoquinoline ring, a carbazole ring, a phenanthridine ring, an acridine ring, a phenanthroline ring, a thianthrene ring, a chromen ring, a xanthene ring, a phenoxathiine ring, a phenothiazine ring, and a phenazine ring).

R^{1'} to R^{13'} are preferably a hydrogen atom, a halogen atom, an alkyl group (including a cycloalkyl group, a bicycloalkyl group, and a tricycloalkyl group), an alkenyl group (including a cycloalkenyl group and a bicycloalkenyl group), an alkynyl group, an aryl group, a cyano group, a hydroxyl group, a carboxyl group, an alkoxy group, an aryloxy group, an acyloxy group, a carbamoyloxy group, an acylamino group, an aminocarbonylamino group, an alkoxycarbonylamino group, an aryloxycarbonylamino group, a sulfamoylamino group, an alkyl or aryl sulfonylamino group, an alkylthio group, an arylthio group, a sulfamoyl group, an alkyl or aryl sulfonyl group, an aryloxycarbonyl group, an alkoxycarbonyl group, a carbamoyl group, an imide group, a silyl group, or a ureido group.

However, at least one of R^{1'} to R^{8'} denotes a halogen atom (fluorine, bromine, chlorine, iodine) or a haloalkyl group (e.g. a trifluoromethyl group, a trichloromethyl, a tribromomethyl group, a perfluorobutyl group, etc.).

R^{9'} to R^{13'} preferably comprise a hydrogen atom, a halogen atom (fluorine, bromine, chlorine, iodine), or a haloalkyl group from the viewpoints of coloration, and stability over time from the two causes of thermal stability and reduction in solubility in a polymerizable compound. The haloalkyl group is preferably a trihaloalkyl group. A halogen atom or a haloalkyl group is incorporated into any one of R^{1'} to R^{8'}, and it is preferable for more than two thereof to be incorporated from the viewpoint of sensitivity. Furthermore, it is more preferable for at least one of R^{1'} to R^{4'} and at least one of R^{5'} to R^{8'} to be a halogen atom or a trihalomethyl group. The sulfonium salt is preferably substituted with a halogen atom, and more preferably with a chlorine atom. Moreover, it is particularly preferable that R^{3'} and R^{6'} are substituted with -Cl.

R^{9'} to R^{13'} are particularly preferably a hydrogen atom, an alkyl group (including a cycloalkyl group, a bicycloalkyl group, and a tricycloalkyl group), a cyano group, a hydroxyl group, an alkoxy group, or an alkylsulfonyl group, provided that preferably not all of R^{9'} to R^{13'} are hydrogen atoms from the viewpoint of benzene not being generated. Furthermore, it is preferable from the viewpoint of sensitivity that they are substituted with at least one halogen atom.

The sulfonium salt having a cation represented by Formula (II) (sulfonium salt (A)) has a counteranion. Due to the counteranion, it is electrically neutral. From the viewpoint of stability, examples of the anion include a sulfonic acid anion (e.g. an alkylsulfonic acid anion or an arylsulfonic acid anion), a benzoylformic acid anion, PF₆⁻, SbF₆⁻, BF₄⁻, ClO₄⁻, a carboxylic acid anion (e.g. an alkylcarboxylic acid anion, an arylcarboxylic acid anion, or an aralkylcarboxylic acid anion), a sulfinic acid anion, a sulfuric acid anion, a borate anion, a sulfonylimide anion, a bis(alkylsulfonyl)imide anion, a tris(alkylsulfonyl)methyl anion, a halogen anion, a polymer-based sulfonic acid anion, and a polymer-based carboxylic acid anion.

Furthermore, the anion is preferably a non-nucleophilic anion. The non-nucleophilic anion referred to here is an anion whose ability to cause a nucleophilic reaction is very low and that can suppress decomposition over time due to an intramolecular nucleophilic reaction.

Preferred examples of the non-nucleophilic anion include PF₆⁻, SbF₆⁻, BF₄⁻, a sulfonylimide anion, a bis(alkylsulfonyl)imide anion, a tris(alkylsulfonyl)methyl anion, and a tetraarylborate anion (e.g. B(C₆F₅)₄⁻).

In the present invention, the sulfonium salt having a cation represented by Formula (II) may be one having a polymer-based anion and a plurality of triarylsulfonium salt structures as countercations.

The alkyl moiety of the alkylsulfonic acid anion may be an alkyl group or a cycloalkyl group, and may preferably be an alkyl group having 1 to 30 carbons or a cycloalkyl group having 3 to 30 carbons; examples thereof include a methyl group, an ethyl group, a propyl group, an isopropyl group, an n-butyl group, an isobutyl group, a sec-butyl group, a pentyl group, a neopentyl group, a hexyl group, a heptyl group, an octyl group, a nonyl group, a decyl group, an undecyl group, a dodecyl group, a tridecyl group, a tetradecyl group, a pentadecyl group, a hexadecyl group, a heptadecyl group, an octadecyl group, a nonadecyl group, an eicosyl group, a cyclopropyl group, a cyclopentyl group, a cyclohexyl group, an adamantyl group, a norbornyl group, and a bornyl group.

The aryl group in the arylsulfonic acid anion is preferably an aryl group having 6 to 14 carbons; examples thereof include a phenyl group, a tolyl group, and a naphthyl group.

With regard to the substituent of the alkyl group, the cycloalkyl group, and the aryl group in the above-mentioned alkylsulfonic acid anion and arylsulfonic acid anion, examples thereof include a nitro group, a halogen atom (a fluorine atom, a chlorine atom, a bromine atom, or an iodine atom), a carboxyl group, a hydroxyl group, an amino group, a cyano group, an alkoxy group (preferably 1 to 5 carbons), a cycloalkyl group (preferably 3 to 15 carbons), an aryl group (preferably 6 to 14 carbons), an alkoxycarbonyl group (preferably 2 to 7 carbons), an acyl group (preferably 2 to 12 carbons), an alkoxycarbonyloxy group (preferably 2 to 7 carbons), and alkylthio group. The aryl group and cyclic structure possessed by each group may further have an alkyl group (preferably 1 to 15 carbons) as a substituent.

With regard to the alkyl moiety of the alkylcarboxylic acid anion, examples thereof include the same alkyl groups and cycloalkyl groups as those cited for the alkylsulfonic acid anion. With regard to the aryl group of the arylcarboxylic acid anion, examples thereof include the same aryl groups as those cited for the arylsulfonic acid anion. With regard to the aralkyl group of the aralkylcarboxylic acid anion, it is preferably an aralkyl group having 6 to 12 carbons, and examples thereof include a benzyl group, a phenethyl group, a naphthylmethyl group, and a naphthylethyl group.

With regard to the substituent of the alkyl group, the cycloalkyl group, the aryl group, and the aralkyl group in the above-mentioned alkylcarboxylic acid anion, arylcarboxylic acid anion, and aralkylcarboxylic acid anion, examples thereof include the same halogen atom, alkyl group, cycloalkyl group, alkoxy group, and alkylthio group as those cited for the arylsulfonic acid anion.

Examples of the sulfonylimide anion include a saccharin anion.

The alkyl group of the bis(alkylsulfonyl)imide anion and the tris(alkylsulfonyl)methyl anion is preferably an alkyl group having 1 to 5 carbons; examples thereof include a methyl group, an ethyl group, a propyl group, an isopropyl group, an n-butyl group, an isobutyl group, a sec-butyl group, a pentyl group, and a neopentyl group. Examples of substituents for these alkyl groups include a halogen atom, and an alkyl group, alkoxy group, and alkylthio group substituted with a halogen atom.

The anion possessed, together with the cation represented by Formula (II), by the sulfonium salt (A) may be monovalent or divalent. When the anion is di- or higher-valent, the sulfonium salt (A) may have two or more cations represented by Formula (II).

The sulfonium salt having a cation represented by Formula (II) (sulfonium salt (A)) may be synthesized by incorporating a halogen into a cyclic sulfide compound by means of a halogenating agent, oxidizing it with hydrogen peroxide to give a sulfoxide, reacting it with a diphenylsulfoxide compound in the presence of an acid catalyst to synthesize a triphenylsulfonium salt structure, and then subjecting it to salt exchange with a desired anion.

The sulfonium salt having a cation represented by Formula (II) is preferably contained at 1 to 30 wt % on a solids content basis in the curable composition, and more preferably at 2 to 20 wt %. It is preferable for the content thereof to be in the above-mentioned range since a sufficient amount of acid is generated and at the same time the solubility is high. The solids content basis referred to here means a proportion expressed as wt % after a volatile component such as a solvent is removed from the curable composition.

Preferred specific examples of the cation represented by Formula (II) are illustrated below, but the present invention is not limited thereby. The sulfonium salt (A) of the present invention has a cation represented by Formula (II) and a counteranion, and is electrically neutral.

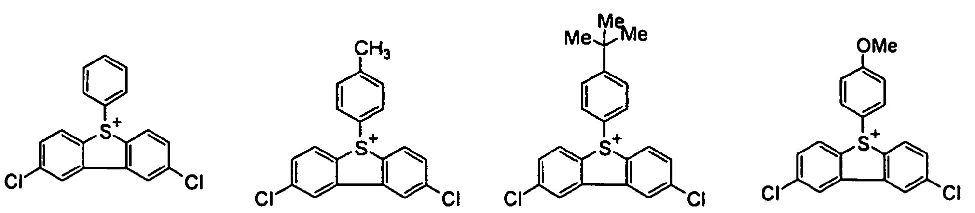

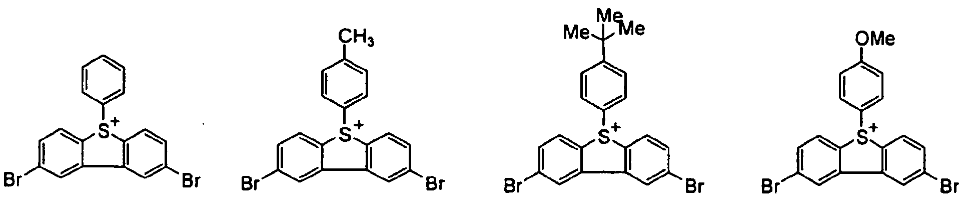

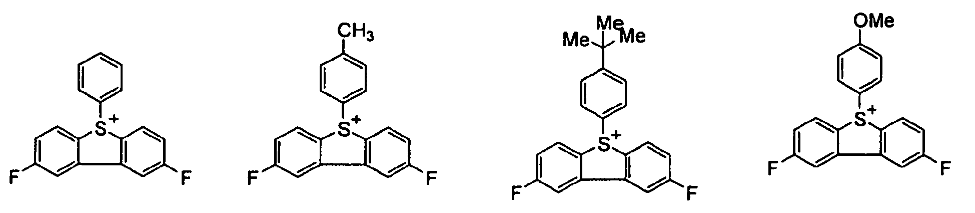

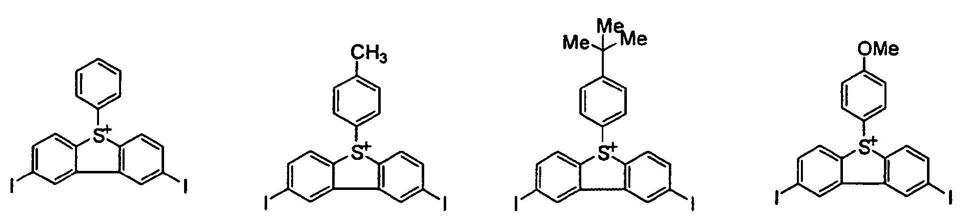

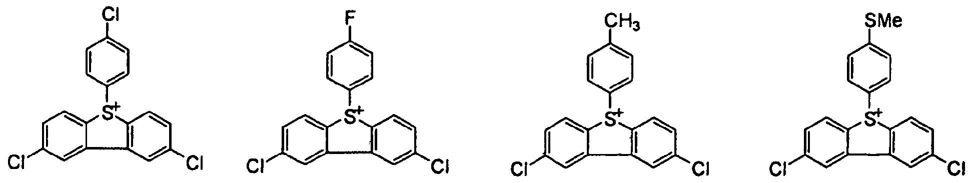

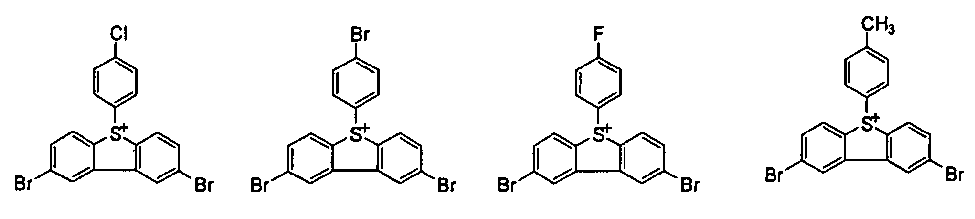

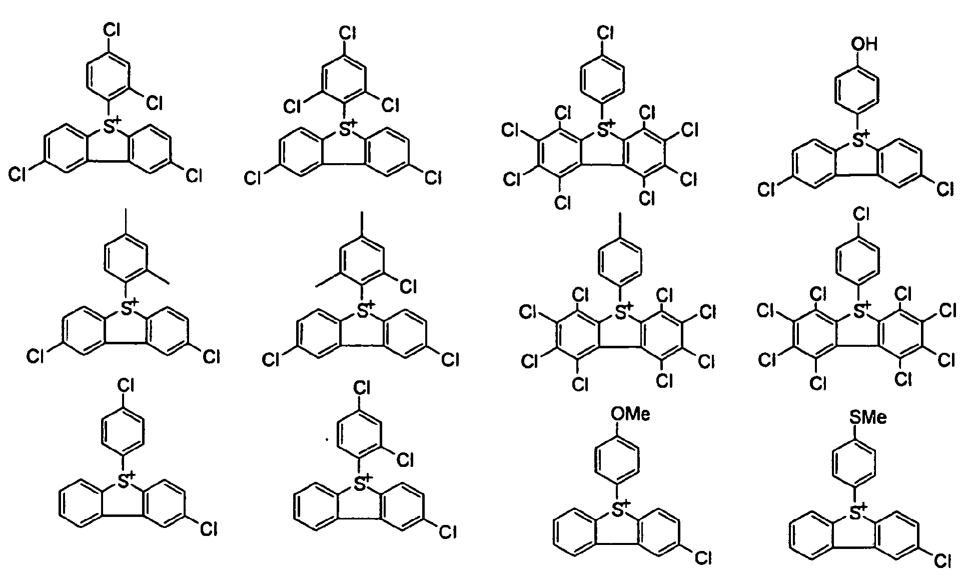

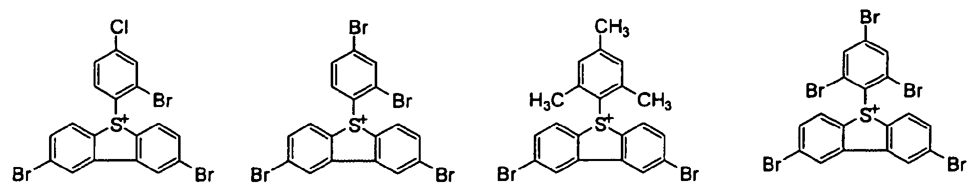

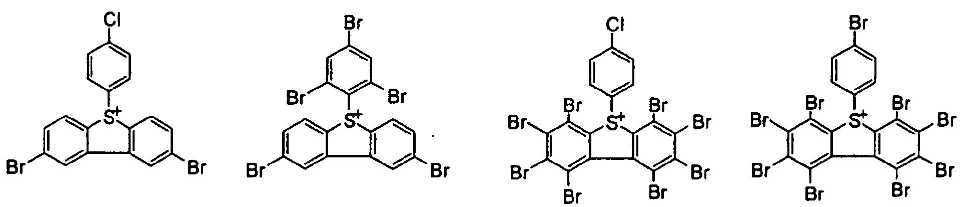

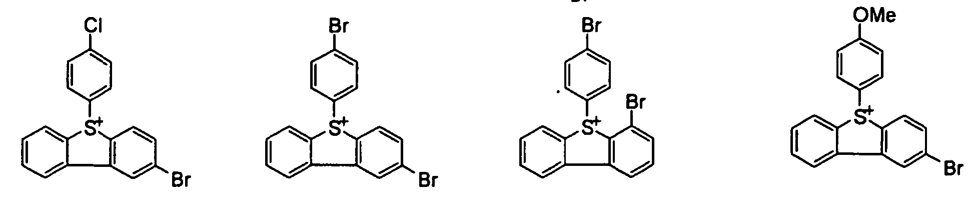

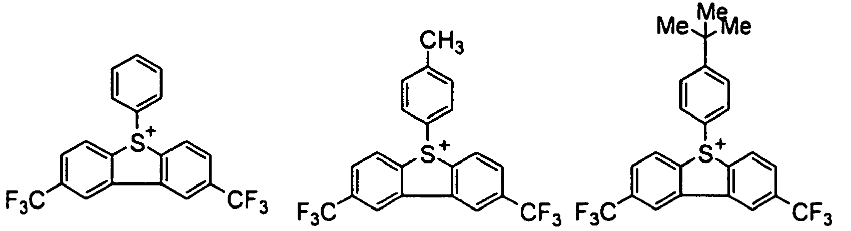

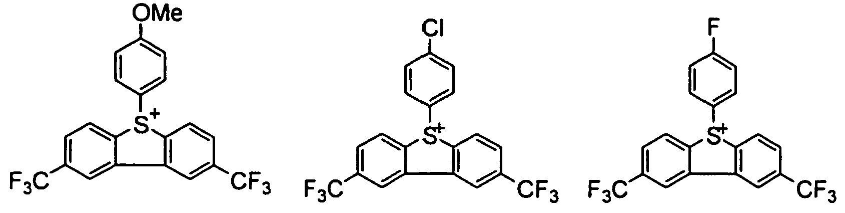

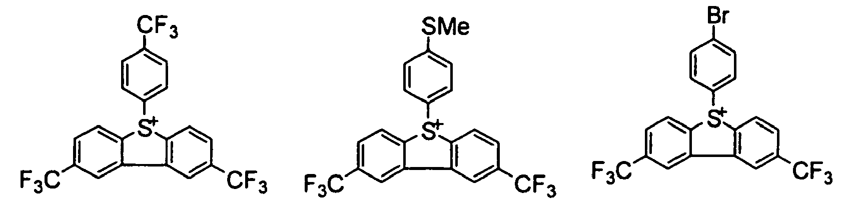

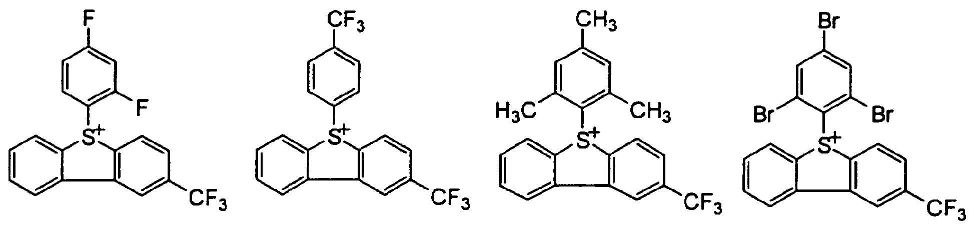

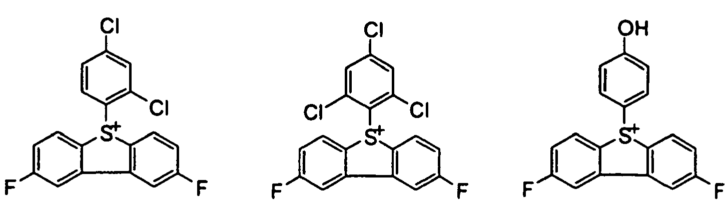

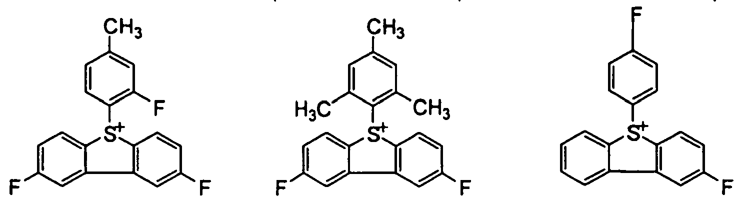

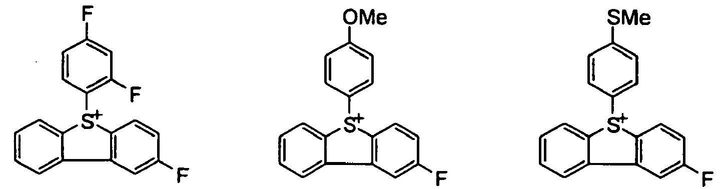

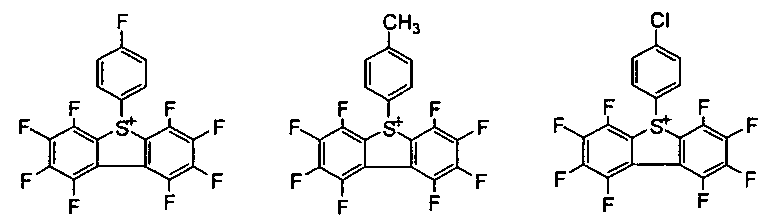

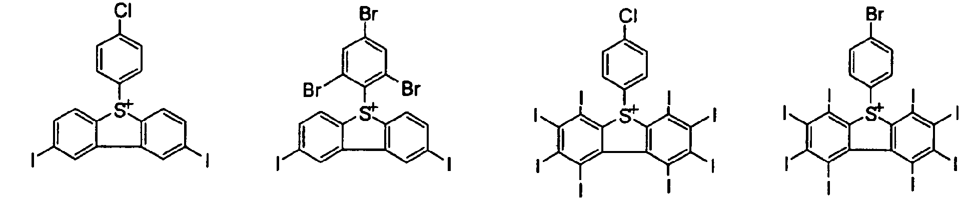

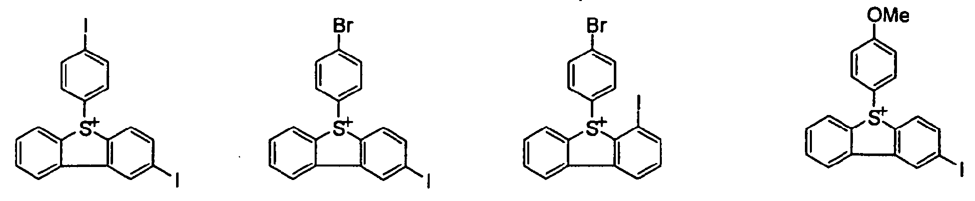

With regard to a counteranion of the cation represented by Formula (II) above, the following anions are preferable.

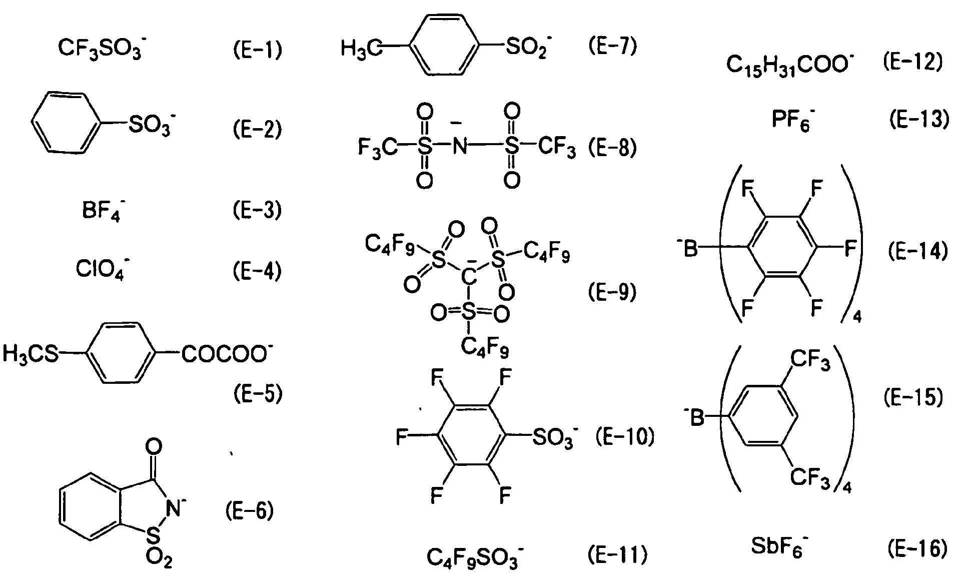

These cations and counteranions may be used in any combination and may be selected appropriately according to a desired purpose.

Among the above, the structures below are particularly preferable.

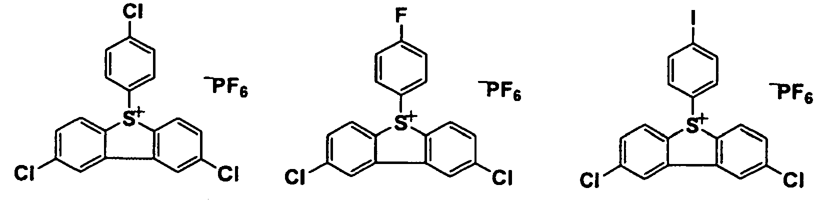

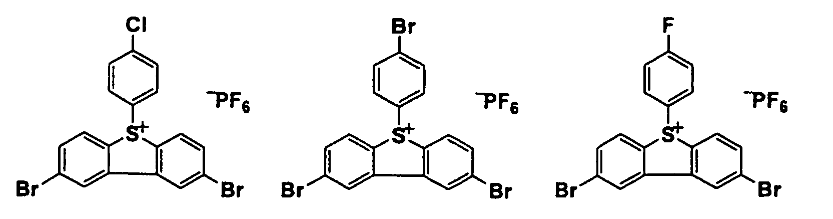

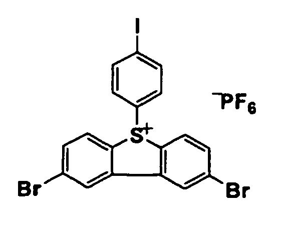

### (1) Curable composition

The curable composition of the present invention can be cured by radiation, and comprises at least (a) the above-mentioned sulfonium salt (A), (b) a polymerizable compound, and (c) a sensitizer.

The 'radiation' referred to in the present invention is not particularly limited as long as it is actinic radiation that can provide energy that enables an initiating species to be generated in the curable composition when irradiated, and broadly includes α rays, γ rays, X rays, ultraviolet rays, visible light, and an electron beam; among these, ultraviolet rays and an electron beam are preferable from the viewpoint of curing sensitivity and the availability of equipment, and ultraviolet rays are particularly preferable. The curable composition of the present invention is therefore preferably a curable composition that can be cured by exposure to ultraviolet rays as radiation.

The curable composition of the present invention may be used in various applications; it can be used suitably in the production of an ink composition, a clear ink (colorless ink composition), a coating agent that imparts surface smoothness, gas barrier properties, gloss, scratch resistance, etc., a lithographic printing plate, a holographic material, a resist, a color filter, and an optical disk, in a production process of a semiconductor such as an IC, and in the production of a circuit board for a liquid crystal display, a thermal head, etc. It is also useful as a modeling material for stereolithography, and it is also useful as a photocuring resin material for a paint, an adhesive, etc.

When the curable composition of the present invention is used as a colorless clear ink, it can be used for a Braille sticker, a Braille display, etc. Furthermore, the curable composition of the present invention is not limited to formation of an image and can be used in a wide range of applications, including, for example, imparting surface smoothness, etc. Specifically, in order to improve the surface smoothness of a recording medium on which an image has been formed with an inkjet ink, the curable composition of the present invention is applied and cured so as to bury irregularities thereof, and the surface can thus be smoothed.

Among these applications, the curable composition of the present invention is preferably used as a clear ink or an ink composition, and particularly preferably as an ink composition. When it is used as an ink composition, it preferably comprises (d) a colorant.

### (b) Polymerizable compound

In the present invention, as the polymerizable compound either a cationically polymerizable compound or a radically polymerizable compound may be used. It is also possible to use a cationically polymerizable compound and a radically polymerizable compound in combination. Furthermore, each of the cationically polymerizable compound and the radically polymerizable compound may be used singly or in a combination of a plurality of types thereof.

Among these, it is preferable to use a cationically polymerizable compound. Furthermore, a cationically polymerizable compound and a radically polymerizable compound may be used in combination. It is preferable to use a cationically polymerizable compound since there is little oxygen inhibition and a high sensitivity is obtained.

When a cationically polymerizable compound and a radically polymerizable compound are used in combination, the ratio by weight of the cationically polymerizable compound to the radically polymerizable compound is preferably 10:90 to 90:10, and more preferably 20:80 to 80:20. It is preferable for the ratio by weight to be in the above-mentioned range since a high film strength can be obtained.

Each of the cationically polymerizable compound and the radically polymerizable compound is explained below.

### (b-1) Cationically polymerizable compound

The cationically polymerizable compound used in the present invention is not particularly limited as long as it is a compound that undergoes a polymerization reaction by virtue of cationic polymerization initiating species generated by a cationic polymerization initiator and is cured, and various types of cationically polymerizable monomers known as photo-cationically polymerizable monomers may be used. Examples of the cationically polymerizable monomer include epoxy compounds, vinyl ether compounds, oxetane compounds described in JP-A-6-9714, JP-A-2001-31892, JP-A-2001-40068, JP-A-2001-55507, JP-A-2001-310938, JP-A-2001-310937, JP-A-2001-220526, etc. Moreover, as the cationically polymerization compound, a cationically photo-curable resin is known, and the photo-cationically polymerizable photo-carable resins that are sensitized to the wavelength of 400nm or greater are described in JP-A-6-43633, JP-A-8-24137, etc, recently.

Examples of the epoxy compounds include aromatic epoxides, alicyclic epoxides, and aliphatic epoxides, and examples of the aromatic epoxide include di- or polyglycidyl ethers produced by a reaction between epichlorohydrin and a polyhydric phenol having at least one aromatic nucleus or an alkylene oxide adduct thereof; specific examples include di- or polyglycidyl ethers of bisphenol A or an alkylene oxide adduct thereof, di- or polyglycidyl ethers of hydrogenated bisphenol A or an alkylene oxide adduct thereof, and novolac type epoxy resins. Examples of the alkylene oxide above include ethylene oxide and propylene oxide.

Examples of the alicyclic epoxides include cyclohexene oxide- and cyclopentene oxide-containing compounds obtained by epoxidizing a compound having at least one cycloalkene ring such as a cyclohexene ring or a cyclopentene ring with an appropriate oxidizing agent such as hydrogen peroxide or a peracid.

Examples of the aliphatic epoxides include di- or polyglycidyl ethers of an aliphatic polyhydric alcohol or an alkylene oxide adduct thereof, and representative examples thereof include diglycidyl ethers of an alkylene glycol such as the diglycidyl ether of ethylene glycol, the diglycidyl ether of propylene glycol, and the diglycidyl ether of 1,6-hexanediol, polyglycidyl ethers of a polyhydric alcohol such as the di- or triglycidyl ether of glycerol or an alkylene oxide adduct thereof, and diglycidyl ethers of a polyalkylene glycol such as the diglycidyl ether of polyethylene glycol or an alkylene oxide adduct thereof and the diglycidyl ether of polypropylene glycol or an alkylene oxide adduct thereof. Examples of the alkylene oxide above include ethylene oxide and propylene oxide.

Monofunctional and polyfunctional epoxy compounds that can be used in the present invention are illustrated in detail.

Examples of monofunctional epoxy compounds used in the present invention include phenyl glycidyl ether, p-tert-butylphenyl glycidyl ether, butyl glycidyl ether, 2-ethylhexyl glycidyl ether, allyl glycidyl ether, 1,2-butylene oxide, 1,3-butadiene monooxide, 1,2-epoxydodecane, epichlorohydrin, 1,2-epoxydecane, styrene oxide, cyclohexene oxide, 3-methacryloyloxymethylcyclohexene oxide, 3-acryloyloxymethylcyclohexene oxide, 3-vinylcyclohexene oxide, and 4-vinylcyclohexene oxide..

Furthermore, examples of polyfunctional epoxy compounds include bisphenol A diglycidyl ether, bisphenol F diglycidyl ether, bisphenol S diglycidyl ether, brominated bisphenol A diglycidyl ether, brominated bisphenol F diglycidyl ether, brominated bisphenol S diglycidyl ether, epoxy novolac resins, hydrogenated bisphenol A diglycidyl ether, hydrogenated bisphenol F diglycidyl ether, hydrogenated bisphenol S diglycidyl ether, 3,4-epoxycyclohexenylmethyl-3',4'-epoxycyclohexenecarboxylate, 2-(3,4-epoxycyclohexyl-5,5-spiro-3,4-epoxy)cyclohexane-meta-dioxane, bis(3,4-epoxycyclohexylmethyl) adipate, vinylcyclohexene dioxide, bis(3,4-epoxy-6-methylcyclohexylmethyl) adipate, 3,4-epoxy-6-methylcyclohexenyl 3',4'-epoxy-6'-methylcyclohexenecarboxylate, methylenebis(3,4-epoxycyclohexane), dicyclopentadiene diepoxide, the di(3,4-epoxycyclohexylmethyl) ether of ethylene glycol, ethylene bis(3,4-epoxycyclohexanecarboxylate), dioctyl epoxyhexahydrophthalate, di-2-ethylhexyl epoxyhexahydrophthalate, 1,4-butanediol diglycidyl ether, 1,6-hexanediol diglycidyl ether, glycerol triglycidyl ether, trimethylolpropane triglycidyl ether, polyethylene glycol diglycidyl ether, polypropylene glycol diglycidyl ether, 1,13-tetradecadiene dioxide, limonene dioxide, 1,2,7,8-diepoxyoctane, and 1,2,5,6-diepoxycyclooctane.

Among these epoxy compounds, the aromatic epoxides and the alicyclic epoxides are preferable from the viewpoint of excellent curing speed, and the alicyclic epoxides are particularly preferable.

Examples of the vinyl ether compounds include di- or tri-vinyl ether compounds such as ethylene glycol divinyl ether, diethylene glycol divinyl ether, triethylene glycol divinyl ether, propylene glycol divinyl ether, dipropylene glycol divinyl ether, butanediol divinyl ether, hexanediol divinyl ether, cyclohexanedimethanol divinyl ether, and trimethylolpropane trivinyl ether, and monovinyl ether compounds such as ethyl vinyl ether, n-butyl vinyl ether, isobutyl vinyl ether, octadecyl vinyl ether, cyclohexyl vinyl ether, hydroxybutyl vinyl ether, 2-ethylhexyl vinyl ether, cyclohexanedimethanol monovinyl ether, n-propyl vinyl ether, isopropyl vinyl ether, isopropenyl vinyl ether, dodecyl vinyl ether, and diethylene glycol monovinyl ether.

Monofunctional vinyl ethers and polyfunctional vinyl ethers are illustrated in detail below.

Specific examples of monofunctional vinyl ethers include methyl vinyl ether, ethyl vinyl ether, propyl vinyl ether, n-butyl vinyl ether, t-butyl vinyl ether, 2-ethylhexyl vinyl ether, n-nonyl vinyl ether, lauryl vinyl ether, cyclohexyl vinyl ether, cyclohexylmethyl vinyl ether, 4-methylcyclohexylmethyl vinyl ether, benzyl vinyl ether, dicyclopentenyl vinyl ether, 2-dicyclopentenoxyethyl vinyl ether, methoxyethyl vinyl ether, ethoxyethyl vinyl ether, butoxyethyl vinyl ether, methoxyethoxyethyl vinyl ether, ethoxyethoxyethyl vinyl ether, methoxypolyethylene glycol vinyl ether, tetrahydrofurfuryl vinyl ether, 2-hydroxyethyl vinyl ether, 2-hydroxypropyl vinyl ether, 4-hydroxybutyl vinyl ether, 4-hydroxymethylcyclohexylmethyl vinyl ether, diethylene glycol monovinyl ether, polyethylene glycol vinyl ether, chloroethyl vinyl ether, chlorobutyl vinyl ether, chloroethoxyethyl vinyl ether, phenylethyl vinyl ether, and phenoxypolyethylene glycol vinyl ether.

Furthermore, examples of polyfunctional vinyl ethers include divinyl ethers such as ethylene glycol divinyl ether, diethylene glycol divinyl ether, polyethylene glycol divinyl ether, propylene glycol divinyl ether, butylene glycol divinyl ether, hexanediol divinyl ether, bisphenol A alkylene oxide divinyl ether, and bisphenol F alkylene oxide divinyl ether; and polyfunctional vinyl ethers such as trimethylolethane trivinyl ether, trimethylolpropane trivinyl ether, ditrimethylolpropane tetravinyl ether, glycerol trivinyl ether, pentaerythritol tetravinyl ether, dipentaerythritol pentavinyl ether, dipentaerythritol hexavinyl ether, an ethylene oxide adduct of trimethylolpropane trivinyl ether, a propylene oxide adduct of trimethylolpropane trivinyl ether, an ethylene oxide adduct of ditrimethylolpropane tetravinyl ether, a propylene oxide adduct of ditrimethylolpropane tetravinyl ether, an ethylene oxide adduct of pentaerythritol tetravinyl ether, a propylene oxide adduct of pentaerythritol tetravinyl ether, an ethylene oxide adduct of dipentaerythritol hexavinyl ether, and a propylene oxide adduct of dipentaerythritol hexavinyl ether.

As the vinyl ether compound, the di- or tri-vinyl ether compounds are preferable from the viewpoint of curability, in particular, when the curable composition of the present invention is used as the ink composition, di- or tri-vinyl compounds are preferable from the viewpoint of adhesion to a recording medium, surface hardness of the image formed, etc., and the divinyl ether compounds are particularly preferable.

The oxetane compound in the present invention means a compound having an oxetane ring, and may be selected freely from known oxetane compounds such as those described in JP-A-2001-220526, JP-A-2001-310937, and JP-A-2003-341217.

As the compound having an oxetane ring that can be used in the ink composition of the present invention, a compound having 1 to 4 oxetane rings in the structure is preferable. In accordance with use of such a compound, it becomes easy to maintain the viscosity of the curable composition in a range that gives good handling properties and, furthermore, when the curable composition is used as the ink composition, the cured ink can be given high adhesion to the recording medium, which is preferable.

Examples of compounds having 1 to 2 oxetane rings in the molecule include compounds represented by Formulae (1) to (3) below.

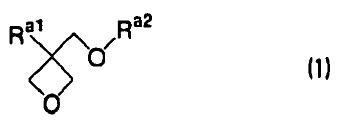

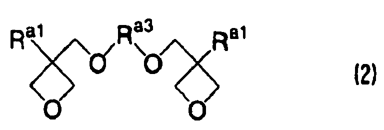

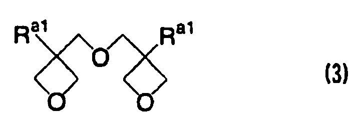

R^{a1} denotes a hydrogen atom, an alkyl group having 1 to 6 carbons, a fluoroalkyl group having 1 to 6 carbons, an allyl group, an aryl group, a furyl group, or a thienyl group. When there are two R^{a1} in the molecule, they may be identical to or different from each other.

Examples of the alkyl group include a methyl group, an ethyl group, a propyl group, and a butyl group, and preferred examples of the fluoroalkyl group include those obtained by substituting any of the hydrogen atoms of the above alkyl groups with a fluorine atom.

R^{a2} denotes a hydrogen atom, an alkyl group having 1 to 6 carbons, an alkenyl group having 2 to 6 carbons, a group having an aromatic ring, an alkylcarbonyl group having 2 to 6 carbons, an alkoxycarbonyl group having 2 to 6 carbons, or an N-alkylcarbamoyl group having 2 to 6 carbons. Examples of the alkyl group include a methyl group, an ethyl group, a propyl group, and a butyl group, examples of the alkenyl group include a 1-propenyl group, a 2-propenyl group, a 2-methyl-1-propenyl group, a 2-methyl-2-propenyl group, a 1-butenyl group, a 2-butenyl group, and a 3-butenyl group, and examples of the group having an aromatic ring include a phenyl group, a benzyl group, a fluorobenzyl group, a methoxybenzyl group, and a phenoxyethyl group. Examples of the alkylcarbonyl group include an ethylcarbonyl group, a propylcarbonyl group, and a butylcarbonyl group, examples of the alkoxycarbonyl group include an ethoxycarbonyl group, a propoxycarbonyl group, and a butoxycarbonyl group, and examples of the N-alkylcarbamoyl group include an ethylcarbamoyl group, a propylcarbamoyl group, a butylcarbamoyl group, and a pentylcarbamoyl group. Furthermore, R^{a2} may have a substituent, and examples of the substituent include a fluorine atom and an alkyl group having 1 to 6 carbons.

R^{a3} denotes a linear or branched alkylene group, a linear or branched poly(alkyleneoxy) group, a linear or branched unsaturated hydrocarbon group, a carbonyl group, a carbonyl group-containing alkylene group, a carboxyl group-containing alkylene group, a carbamoyl group-containing alkylene group, or a group shown below. Examples of the alkylene group include an ethylene group, a propylene group, and a butylene group, and examples of the poly(alkyleneoxy) group include a poly(ethyleneoxy) group and a poly(propyleneoxy) group. Examples of the unsaturated hydrocarbon group include a propenylene group, a methylpropenylene group, and a butenylene group.

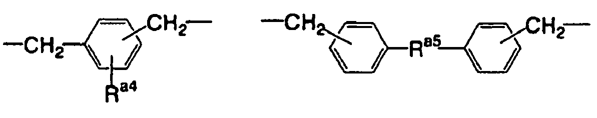

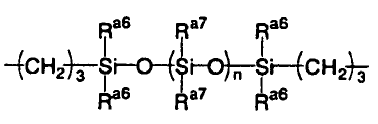

When R^{a3} is the above-mentioned polyvalent group, R^{a4} denotes a hydrogen atom, an alkyl group having 1 to 4 carbons, an alkoxy group having 1 to 4 carbons, a halogen atom, a nitro group, a cyano group, a mercapto group, a lower alkylcarboxyl group, a carboxyl group, or a carbamoyl group.

R^{a5} denotes an oxygen atom, a sulfur atom, a methylene group, NH, SO, SO₂, C(CF₃)₂, or C(CH₃)₂.

R^{a6} denotes an alkyl group having 1 to 4 carbons or an aryl group, and n is an integer of 0 to 2,000. R^{a7} denotes an alkyl group having 1 to 4 carbons, an aryl group, or a monovalent group having the structure below. In the formula, R^{a8} denotes an alkyl group having 1 to 4 carbons or an aryl group, and m is an integer of 0 to 100.

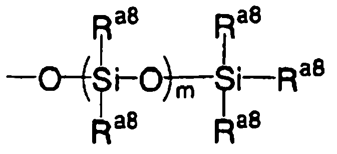

Examples of the compound represented by Formula (1) include 3-ethyl-3-hydroxymethyloxetane (OXT-101: manufactured by Toagosei Co., Ltd.), 3-ethyl-3-(2-ethylhexyloxymethyl)oxetane (OXT-212: manufactured by Toagosei Co., Ltd.), and 3-ethyl-3-phenoxymethyloxetane (OXT-211: manufactured by Toagosei Co., Ltd.). Examples of the compound represented by Formula (2) include 1,4-bis[(3-ethyl-3-oxetanylmethoxy)methyl]benzene (OXT-121: Toagosei Co., Ltd.). Examples of the compound represented by Formula (3) include bis(3-ethyl-3-oxetanylmethyl) ether (OXT-221: Toagosei Co., Ltd.).

Examples of the compound having 3 to 4 oxetane rings in the molecule include compounds represented by Formula (4) below.

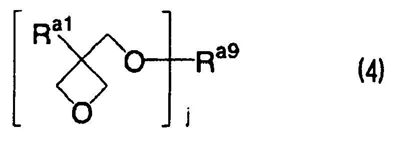

In Formula (4), R^{a1} denotes the same as in Formula (1) above. Furthermore, examples of R^{a9}, which is a polyvalent linking group, include a branched alkylene group having 1 to 12 carbons such as a group represented by A to C below, a branched poly(alkyleneoxy) group such as a group represented by D below, and a branched polysiloxane group such as a group represented by E below. j is 3 or 4.

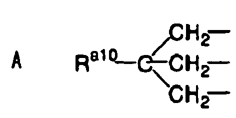

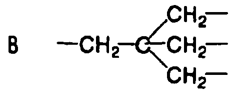

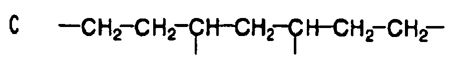

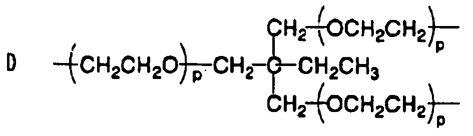

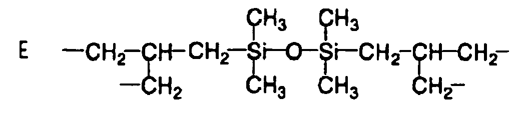

In the above A, R^{a10} denotes a methyl group, an ethyl group, or a propyl group. Furthermore, in the above D, p is an integer of 1 to 10.

Moreover, as another embodiment of the oxetane compound that can be suitably used in the present invention, a compound having an oxetane ring on a side chain, represented by Formula (5) below, can be cited.

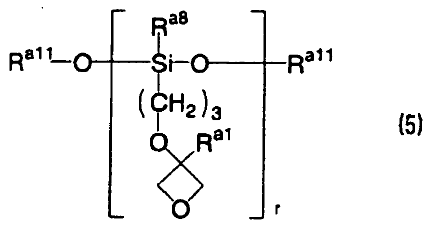

In Formula (5), R^{a1} and R^{a8} denote the same as in the above-mentioned formula. R^{a11} is an alkyl group having 1 to 4 carbons such as a methyl group, an ethyl group, a propyl group, or a butyl group, or a trialkylsilyl group, and r is 1 to 4.

Such compounds having an oxetane ring are described in detail in paragraph Nos. 0021 to 0084 of JP-A-2003-341217 above, and the compounds described here may be suitably used in the present invention.

Furthermore, oxetane compounds described in JP-A-2004-91556 can also be used in the present invention. They are described in detail in Paragraph Nos. 0022 to 0058.

Among the oxetane compounds used in the present invention, from the viewpoint of curable composition viscosity and tackiness, it is preferable to use a compound having one oxetane ring.

The curable composition of the present invention may comprise only one type of cationically polymerizable compound or two or more types thereof in combination, but from the viewpoint of suppressing effectively shrinkage during curing, it is preferable to use a combination of a vinyl ether compound and at least one type of compound selected from the oxetane compounds and the epoxy compounds.

### (b-2) Radically polymerizable compound

In the present invention, as a polymerizable compound a radically polymerizable compound may also be used.

The radically polymerizable compound referred to here is a radically polymerizable organic compound that undergoes a polymerization or crosslinking reaction upon irradiation with actinic radiation in the presence of a radical polymerization initiator, and is preferably a compound having at least one ethylenically unsaturated double bond per molecule.

As the radically polymerizable compound, photocuring materials employing a photopolymerizable composition described in, for example, JP-A-7-159983, JP-B-7-31399, JP-A-8-224982, JP-A-10-863, JP-A-9-80675, etc. are known.

Preferred examples of such a compound include an acrylate compound, a methacrylate compound, an allylurethane compound, an unsaturated polyester compound, and a styrene-based compound.

Among such radically polymerizable compounds, a compound having a (meth)acrylic group is preferable since it is easy to synthesize and obtain, and the handling is also easy. Examples thereof include an epoxy (meth)acrylate, a urethane (meth)acrylate, a polyester (meth)acrylate, a polyether (meth)acrylate, and a (meth)acrylic acid ester of an alcohol.

(Meth)acrylic acid denotes acrylic acid, methacrylic acid, or a mixture thereof, and (meth)acrylate denotes an acrylate, a methacrylate, or a mixture thereof.

The epoxy (meth)acrylate referred to here is, for example, a (meth)acrylate obtained by reacting (meth)acrylic acid with a conventionally known aromatic epoxy resin, alicyclic epoxy resin, aliphatic epoxy resin, etc.

Among these epoxy acrylates, an acrylate of an aromatic epoxy resin is particularly preferable, and it is a (meth)acrylate obtained by reacting (meth)acrylic acid with a polyhydric phenol having at least one aromatic nucleus or a polyglycidyl ether of an alkylene oxide adduct thereof. Examples thereof include a (meth)acrylate obtained by reacting (meth)acrylic acid with a glycidyl ether obtained by reacting bisphenol A or an alkylene oxide adduct thereof with epichlorohydrin, and a (meth)acrylate obtaining by reacting an epoxy novolac resin with (meth)acrylic acid.

The urethane (meth)acrylate is preferably a (meth)acrylate obtained by reacting one or more types of hydroxyl group-containing polyester or hydroxyl group-containing polyether with a hydroxyl group-containing (meth)acrylic acid ester and an isocyanate, or a (meth)acrylate obtained by reacting a hydroxyl group-containing (meth)acrylic acid ester with an isocyanate, etc.

A preferred hydroxyl group-containing polyester used here is a hydroxyl group-containing polyester obtained by a reaction between one or more types of polyhydric alcohol and one or more types of polybasic acid, and examples of aliphatic polyhydric alcohols include 1,3-butanediol, 1,4-butanediol, 1,6-hexanediol, diethylene glycol, triethylene glycol, neopentyl glycol, polyethylene glycol, polypropylene glycol, polybutylene glycol, trimethylolpropane, glycerol, pentaerythritol, and dipentaerythritol. Examples of the polybasic acid include adipic acid, terephthalic acid, phthalic anhydride, and trimellitic acid.

A preferred hydroxyl group-containing polyether is a hydroxyl group-containing polyether obtained by adding one or more types of alkylene oxide to a polyhydric alcohol, and examples of the polyhydric alcohol include the same compounds as those described above. Examples of the alkylene oxide include ethylene oxide, propylene oxide, and butylene oxide.

The hydroxyl group-containing (meth)acrylic acid ester is preferably a hydroxyl group-containing (meth)acrylic acid ester obtained by an esterification reaction between a polyhydric alcohol and (meth)acrylic acid, and examples of the polyhydric alcohol include the same compounds as those described above.

Among such hydroxyl group-containing (meth)acrylic acid esters, a hydroxyl group-containing (meth)acrylic acid ester obtained by an esterification reaction between a divalent alcohol and (meth)acrylic acid is particularly preferable, and examples thereof include 2-hydroxyethyl (meth)acrylate.

As the isocyanate, a compound having at least one isocyanate group per molecule is preferable, and divalent isocyanate compounds such as tolylene diisocyanate, hexamethylene diisocyanate, and isophorone diisocyanate are particularly preferable.

The polyester (meth)acrylate is preferably a polyester (meth)acrylate obtained by a reaction between a hydroxyl group-containing polyester and (meth)acrylic acid.

The hydroxyl group-containing polyester used here is preferably a hydroxyl group-containing polyester obtained by an esterification reaction between one or more types of polyhydric alcohol and one or more types of monobasic acid or polybasic acid, and examples of the polyhydric alcohol include the same compounds as those described above. Examples of the monobasic acid include formic acid, acetic acid, butyric acid, and benzoic acid. Examples of the polybasic acid include adipic acid, terephthalic acid, phthalic anhydride, and trimellitic acid.

The polyether (meth)acrylate is preferably a polyether (meth)acrylate obtained by a reaction between a hydroxyl group-containing polyether and (meth) acrylic acid. The hydroxyl group-containing polyether used here is preferably a hydroxyl group-containing polyether obtained by adding one or more types of alkylene oxide to a polyhydric alcohol, and examples of the polyhydric alcohol include the same compounds as those described above. Examples of the alkylene oxide include ethylene oxide, propylene oxide, and butylene oxide.

The (meth)acrylic acid ester of an alcohol is preferably a (meth)acrylate obtained by reacting an aromatic or aliphatic alcohol having at least one hydroxyl group per molecule or an alkylene oxide adduct thereof with (meth)acrylic acid; examples thereof include 2-ethylhexyl (meth)acrylate, 2-hydroxyethyl (meth)acrylate, 2-hydroxypropyl (meth)acrylate, isoamyl (meth)acrylate, lauryl (meth)acrylate, stearyl (meth)acrylate, isooctyl (meth)acrylate, tetrahydrofurfuryl (meth)acrylate, isobornyl (meth)acrylate, benzyl (meth)acrylate, 1,3-butanediol di(meth)acrylate, 1,4-butanediol di(meth)acrylate, 1,6-hexanediol di(meth)acrylate, diethylene glycol di(meth)acrylate, triethylene glycol di(meth)acrylate, neopentyl glycol di(meth)acrylate, polyethylene glycol di(meth)acrylate, polypropylene glycol di(meth)acrylate, trimethylolpropane tri(meth)acrylate, ethylene oxide-modified trimethylolpropane tri(meth)acrylate, propylene oxide-modified trimethylolpropane tri(meth)acrylate, pentaerythritol tetra(meth)acrylate, dipentaerythritol hexa(meth)acrylate, and ε-caprolactone-modified dipentaerythritol hexa(meth)acrylate. These radically polymerizable compounds may be used singly or in a combination of two or more types depending on a desired performance.

It is preferable for at least 50 parts by weight out of 100 parts by weight of the radically polymerizable compound to be a compound having a (meth)acrylic group in the molecule.

### (c) Sensitizer

In the present invention, the curable composition and the ink composition preferably comprise a sensitizer for the purpose of improving the acid generating efficiency of the photo-acid generator and increasing the photosensitive wavelength. As the sensitizer, one that sensitizes a photo-acid generator by virtue of an electron transfer mechanism or an energy transfer mechanism is preferable.

Preferred examples of the sensitizer include those belonging to the types of compounds below and having an absorption wavelength in the 350 nm to 450 nm region.

Examples include polynuclear aromatic compounds (e.g. phenanthrene, anthracene, pyrene, perylene, triphenylene, a 9,10-dialkoxyanthracene), triphenylamines, xanthenes (e.g. fluorescein, eosin, erythrosine, rhodamine B, rose bengal), thioxanthones (isopropylthioxanthone, diethylthioxanthone, chlorothioxanthone), cyanines (e.g. thiacarbocyanine, oxacarbocyanine), merocyanines (e.g. merocyanine, carbomerocyanine), phthalocyanines, thiazines (e.g. thionine, methylene blue, toluidine blue), acridines (e.g. acridine orange, chloroflavin, acriflavin), anthraquinones (e.g. anthraquinone), squaliums (e.g. squalium), coumarins (e.g. 7-diethylamino-4-methylcoumarin), ketocoumarin, phenothiazines, phenazines, styrylbenzenes, diphenylbutadiene, azo compounds, diphenylmethane, triphenylmethane, distyrylbenzenes, carbazoles, porphyrin, spiro compounds, quinacridone, indigo, styryl compounds, pyrylium compounds, pyrromethene compounds, pyrazolotriazole compounds, benzothiazole compounds, barbituric acid derivatives, thiobarbituric acid derivatives, and compounds described in EP No. 568,993, US Pat. Nos. 4,508,811 and 5,227,227, JP-A-2001-125255, JP-A-11-271969, etc.

Among them, the sulfonium salt of the present invention is preferably combined with a polynuclear aromatic compound (e.g. phenanthrene, anthracene, pyrene, perylene, triphenylene, a 9,10-dialkoxyanthracene), a thioxanthone, a distyrylbenzene, a styrylbenzene, a carbazole or a diphenylbutadiene from the viewpoint of initiation efficiency, and most preferably with a polynuclear aromatic compound, a distyrylbenzene, a styrylbenzene, or a diphenylbutadiene.

Examples of the sensitizer that can be used preferably in the present invention are illustrated below, but the present invention is not limited thereby.

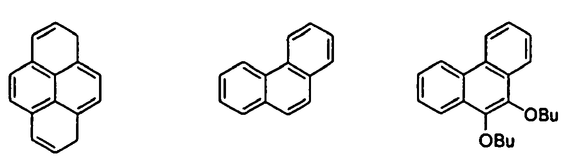

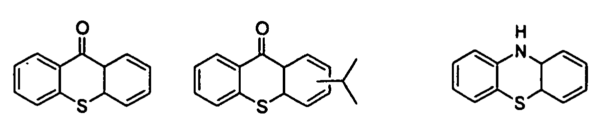

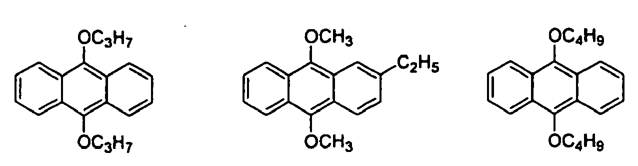

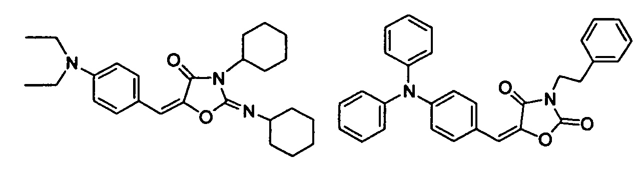

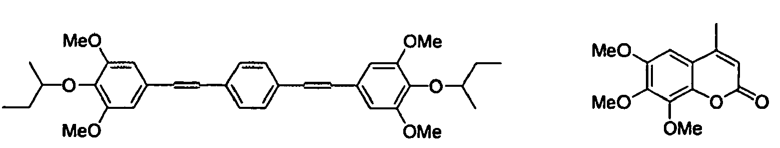

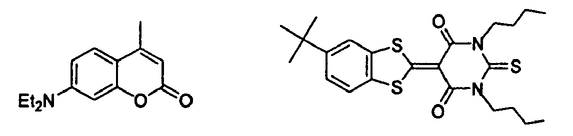

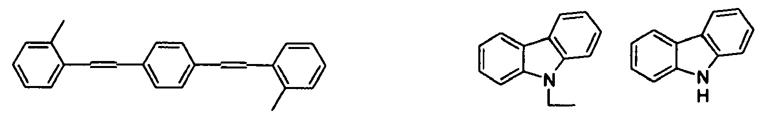

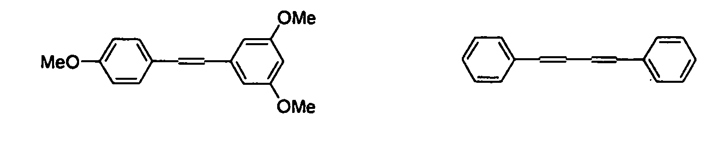

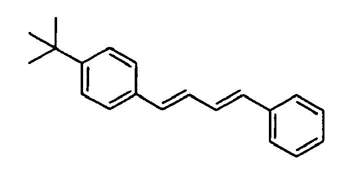

From the viewpoint of coloration, etc. of the curable composition or the ink composition, the content of the sensitizer (c) in the curable composition or the ink composition of the present invention is preferably 0.01 to 20 wt % relative to the entire weight of the curable composition or the ink composition, more preferably 0.1 to 15 wt %, and yet more preferably 0.5 to 10 wt %.

The sensitizer (c) may be used singly or in a combination of two or more types.

### (2) Ink composition

The curable composition of the present invention may be used suitably in an ink composition. That is, in the present invention, the ink composition comprises (a) a sulfonium salt having a cation represented by Formula (II), (b) a polymerizable compound, and (c) a sensitizer, and may comprise as necessary (d) a colorant, (e) a cosensitizer, (f) another polymerization initiator, and (g) another component.

When used as an ink composition, the content of the sulfonium salt (A) is preferably 0.1 to 20 wt % relative to the weight of the entire ink composition, more preferably 0.5 to 10 wt %, and yet more preferably 1 to 7 wt %.

Furthermore, the content of the polymerizable compound (b) in the ink composition is preferably 10 to 95 wt % relative to the weight of the entire ink composition, more preferably 30 to 90 wt %, and yet more preferably 50 to 85 wt %.

### (d) Coloring agent

The coloring agent that can be used in the present invention is not particularly limited, but a pigment and an oil-soluble dye that have excellent weather resistance and rich color reproduction are preferable, and it may be selected from any known coloring agent such as a soluble dye. It is preferable that the coloring agent that can be suitably used in the ink composition or the inkjet recording ink composition of the present invention does not function as a polymerization inhibitor in a polymerization reaction, which is a curing reaction. This is because the sensitivity of the curing reaction by actinic radiation should not be degraded.

### (d-1) Pigment

The pigment that can be used in the present invention is not particularly limited and, for example, organic and inorganic pigments having the numbers below described in the Color Index may be used.

That is, as a red or magenta pigment, Pigment Red 3, 5, 19, 22, 31, 38, 43, 48:1, 48:2, 48:3, 48:4, 48:5, 49:1, 53:1, 57:1, 57:2, 58:4, 63:1, 81, 81:1, 81:2, 81:3, 81:4, 88, 104, 108, 112, 122, 123, 144, 146, 149, 166, 168, 169, 170, 177, 178, 179, 184, 185, 208, 216, 226, or 257, Pigment Violet 3, 19, 23, 29, 30, 37, 50, or 88, and Pigment Orange 13, 16, 20, or 36; as a blue or cyan pigment, Pigment Blue 1, 15, 15:1, 15:2, 15:3, 15:4, 15:6, 16, 17-1, 22, 27, 28, 29, 36, or 60; as a green pigment, Pigment Green 7, 26, 36, or 50; as a yellow pigment, Pigment Yellow 1, 3, 12, 13, 14, 17, 34, 35, 37, 55, 74, 81, 83, 93, 94, 95, 97, 108, 109, 110, 137, 138, 139, 153, 154, 155, 157, 166, 167, 168, 180, 185, or 193; as a black pigment, Pigment Black 7, 28, or 26; as a white pigment, Pigment White 6, 18, or 21, etc. may be used according to the intended application.

The white pigment may impart a white color to the ink composition and can employ a white pigment that is normally used in this field, and as such a white pigment it is preferable to use an inorganic white pigment, an organic white pigment, or white hollow polymer microparticles.

Examples of the inorganic white pigment include an alkaline earth metal sulfate such as barium sulfate, an alkaline earth metal carbonate such as calcium carbonate, a silica such as a fine particle silicic acid or a synthetic silicate, calcium silicate, alumina, alumina hydrate, titanium oxide, zinc oxide, talc, and clay. In the present invention, titanium oxide is particularly preferable from the viewpoint of hiding power, coloration, and dispersion particle size.

Examples of the organic white pigment include an organic compound salt described in JP-A-11-129613 and alkylene bismelamine derivatives described in JP-A-11-140365 and JP-A-2001-234093.

Specific commercial products of the white pigment include Shigenox OWP, Shigenox OWPL, Shigenox FWP, Shigenox FWG, Shigenox UL, and Shigenox U (product names, manufactured by Hakkol Chemical Co., Ltd.).

Examples of the white hollow polymer microparticles include thermoplastic microparticles formed substantially from an organic polymer, disclosed in US Pat. No. 4,089,800. The white pigments may be used singly or in combination.

The white ink may employ a fluorescent whitening agent in combination. As the fluorescent whitening agent, various types of fluorescent whitening agent known to a person skilled in the art may be used; examples thereof include benzoxazole type, coumarin type, and pyrazoline type fluorescent whitening agents, and benzooxazolylnaphthalene type and benzooxazolylstilbene type fluorescent whitening agents are preferable. Examples of the fluorescent whitening agent include those described in JP-A-59-42993, JP-A-59-52689, JP-A-62-280069, JP-A-61-242871, and JP-A-4-219266.

### (d-2) Oil-soluble dye

The oil-soluble dye that can be used in the present invention is explained below.

The oil-soluble dye that can be used in the present invention means a dye that is substantially insoluble in water. Specifically, the solubility in water at 25°C (the mass of dye that can be dissolved in 100 g of water) is no greater than 1 g, preferably no greater than 0.5 g, and more preferably no greater than 0.1 g. Therefore, the oil-soluble dye means a so-called water-insoluble pigment or an oil-soluble dye, and among these the oil-soluble dye is preferable.

In the present invention, the oil-soluble dye may be used singly or in a combination of two or more types. Furthermore, another colorant such as a water-soluble dye, a disperse dye, or a pigment may be contained as necessary in a range that does not interfere with the effects of the present invention.

Among the oil-soluble dyes that can be used in the present invention, as a yellow dye, any may be used. Examples thereof include aryl or heteryl azo dyes having a coupling component such as a phenol, a naphthol, an aniline, a pyrazolone, a pyridone, or an open-chain active methylene compound; azomethine dyes having a coupling component such as an open-chain active methylene compound; methine dyes such as benzylidene dyes and monomethineoxonol dyes; quinone dyes such as naphthoquinone dyes and anthraquinone dyes; and other dye species such as quinophthalone dyes, nitro/nitroso dyes, acridine dyes, and acridinone dyes.

Among the above-mentioned oil-soluble dyes that can be used in the present invention, as a magenta dye, any may be used. Examples thereof include aryl or heteryl azo dyes having a coupling component such as a phenol, a naphthol, or an aniline; azomethine dyes having a coupling component such as a pyrazolone or a pyrazolotriazole; methine dyes such as arylidene dyes, styryl dyes, merocyanine dyes, and oxonol dyes; carbonium dyes such as diphenylmethane dyes, triphenylmethane dyes, and xanthene dyes; quinone dyes such as naphthoquinones, anthraquinones, or anthrapyridones; and condensed polycyclic dyes such as dioxazine dyes.

Among the oil-soluble dyes that can be used in the present invention, as a cyan dye, any may be used. Examples thereof include indoaniline dyes, indophenol dyes, and azomethine dyes having a coupling component such as a pyrrolotriazole; polymethine dyes such as cyanine dyes, oxonol dyes, and merocyanine dyes; carbonium dyes such as diphenylmethane dyes, triphenylmethane dyes, and xanthene dyes; phthalocyanine dyes; anthraquinone dyes; aryl or heteryl azo dyes having a coupling component such as a phenol, a naphthol, or an aniline; and indigo/thioindigo dyes.

The above-mentioned dyes may be dyes that exhibit respective colors of yellow, magenta, and cyan only after a part of the chromophore dissociates, and in that case the counter cation may be an inorganic cation such as an alkali metal or ammonium, may be an organic cation such as pyridinium or a quaternary ammonium salt, or may be a polymer cation having the above cation as a partial structure.

Although not limited to the following, preferred specific examples thereof include CI Solvent Black 3, 7, 27, 29, and 34; CI Solvent Yellow 14, 16, 19, 29, 30, 56, 82, 93, and 162; Cl Solvent Red 1, 3, 8, 18, 24, 27, 43, 49, 51, 72, 73, 109, 122, 132, and 218; Cl Solvent Violet 3; Cl Solvent Blue 2, 11, 25, 35, 38, 67, and 70; Cl Solvent Green 3 and 7; and Cl Solvent Orange 2. Particularly preferred examples thereof include Nubian Black PC-0850, Oil Black HBB, Oil Yellow 129, Oil Yellow 105, Oil Pink 312, Oil Red 5B, Oil Scarlet 308, Vali Fast Blue 2606, Oil Blue BOS (manufactured by Orient Chemical Industries, Ltd.), Aizen Spilon Blue GNH (manufactured by Hodogaya Chemical Co., Ltd.), Neopen Yellow 075, Neopen Magenta SE1378, Neopen Blue 808, Neopen Blue FF4012, and Neopen Cyan FF4238 (manufactured by BASF).

In the present invention, a disperse dye may be used in a range that enables it to be dissolved in a water-immiscible organic solvent. Specific preferred examples thereof include Cl Disperse Yellow 5, 42, 54, 64, 79, 82, 83, 93, 99, 100, 119, 122, 124, 126, 160, 184:1, 186, 198, 199, 201, 204, 224, and 237; Cl Disperse Orange 13, 29, 31:1, 33, 49, 54, 55, 66, 73, 118, 119, and 163; Cl Disperse Red 54, 60, 72, 73, 86, 88, 91, 92, 93, 111, 126, 127, 134, 135, 143, 145, 152, 153, 154, 159, 164, 167:1, 177, 181, 204, 206, 207, 221, 239, 240, 258, 277, 278, 283, 311, 323, 343, 348, 356, and 362; Cl Disperse Violet 33; Cl Disperse Blue 56, 60, 73, 87, 113, 128, 143, 148, 154, 158, 165, 165:1, 165:2, 176, 183, 185, 197, 198, 201, 214, 224, 225, 257, 266, 267, 287, 354, 358, 365, and 368; and Cl Disperse Green 6:1 and 9.

Particularly preferred examples of the oil-soluble dye include azo and azomethine dyes represented by Formulae (1) and (2) below. Dyes represented by Formula (2) below are known, in the photographic material area, as dyes that are generated from a coupler and a developing agent by oxidation.

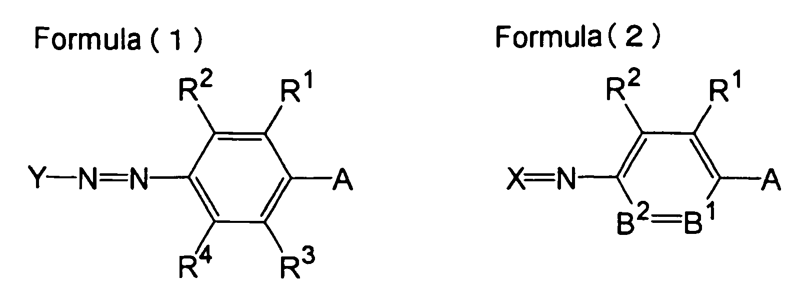

In Formulae (1) and (2) above, R¹, R², R³ and R⁴ independently denote a hydrogen atom, a halogen atom, an aliphatic group, an aromatic group, a heterocyclic group, a cyano group, a hydroxyl group, a nitro group, an amino group, an alkylamino group, an alkoxy group, an aryloxy group, an amide group, an arylamino group, a ureido group, a sulfamoylamino group, an alkylthio group, an arylthio group, an alkoxycarbonylamino group, a sulfonamide group, a carbamoyl group, a sulfamoyl group, a sulfonyl group, an alkoxycarbonyl group, a heterocyclooxy group, an azo group, an acyloxy group, a carbamoyloxy group, a silyloxy group, an aryloxycarbonyl group, an aryloxycarbonylamino group, an imide group, a heterocyclothio group, a sulfinyl group, a phosphoryl group, an acyl group, a carboxyl group, or a sulfo group.

In Formulae (1) and (2) above, in particular, R² is preferably, among the above-mentioned substituents, a hydrogen atom, a halogen atom, an aliphatic group, an alkoxy group, an aryloxy group, an amide group, a ureido group, a sulfamoylamino group, an alkoxycarbonylamino, or a sulfonamide group.

In the present specification, the aliphatic group denotes an alkyl group, a substituted alkyl group, an alkenyl group, a substituted alkenyl group, an alkynyl group, a substituted alkynyl group, an aralkyl group, or a substituted aralkyl group. The aliphatic group may have a branch or form a ring. The number of carbon atoms of the aliphatic group is preferably 1 to 20, and more preferably 1 to 18. The aryl moiety of the aralkyl group and the substituted aralkyl group is preferably phenyl or naphthyl, and particularly preferably phenyl. Examples of the substituents of the alkyl moieties of the substituted alkyl group, the substituted alkenyl group, the substituted alkynyl group, and the substituted aralkyl group include the substituents cited for explanation of R¹ to R⁴. Examples of the substituents of the aryl moiety of the substituted aralkyl group are the same as those of the substituent of the substituted aryl group below.

In the present specification, the aromatic group means an aryl group and a substituted aryl group. The aryl group is preferably phenyl or naphthyl, and particularly preferably phenyl. The aryl moiety of the substituted aryl group is the same as that of the above-mentioned aryl group. Examples of the substituent of the substituted aryl group include substituents cited for explanation of R¹ to R⁴.

In Formulae (1) and (2) above, A denotes -NR⁵R⁶ or a hydroxyl group, and R⁵ and R⁶ independently denote a hydrogen atom, an aliphatic group, an aromatic group, or a heterocyclic group. A is preferably -NR⁵R⁶. R⁵ and R⁶ may be bonded together to form a ring. R⁵ and R⁶ preferably each denote a hydrogen atom, an alkyl group, a substituted alkyl group, an aryl group, or a substituted aryl group, and most preferably a hydrogen atom, an alkyl group having 1 to 18 carbon atoms, or a substituted alkyl group having 1 to 18 carbon atoms.

In Formula (2) above, B¹ denotes =C(R³)- or =N-, and B² denotes -C(R⁴)= or -N=. It is preferable that B¹ and B² are not -N= at the same time, and it is more preferable that B¹ is =C(R³)- and B² is -C(R⁴)=. Any of R¹ and R⁵, R³ and R⁶, and R¹ and R² may be bonded together to form an aromatic ring or a hetero ring.

In Formula (1) above, Y denotes an unsaturated heterocyclic group. Y is preferably a five-membered or six-membered unsaturated hetero ring. The hetero ring may be condensed with an aliphatic ring, an aromatic ring, or another hetero ring. Examples of the hetero atom of the hetero ring include N, O, and S.

Preferred examples of the above-mentioned unsaturated hetero ring include a pyrazole ring, an imidazole ring, a thiazole ring, an isothiazole ring, a thiadiazole ring, a thiophene ring, a benzothiazole ring, a benzoxazole ring, a benzoisothiazole ring, a pyrimidine ring, a pyridine ring, and a quinoline ring. It is also possible for the unsaturated heterocyclic group to have a substituent cited above as R¹ to R⁴.

In Formula (2) above, X denotes a color photographic coupler residue. Preferred examples of the color photographic coupler residue are as follows.

Yellow couplers: US Pat. Nos. 3,933,501, 4,022,620, 4,326,024, and 4,401,752, couplers represented by Formulae (I) and (II) in US Pat. No.4,248,961, JP-B-58-10739, GB Pat. Nos. 1,425,020 and 1,476,760, US. Pat. Nos. 3,973,968, 4,314,023, and 4,511,649, and EP Pat. Nos. 249,473A and 502,424A; couplers represented by Formulae (1) and (2) in EP Pat. No. 513,496A (in particular, Y-28 on page 18); couplers represented by Formula (I) of Claim 1 in EP Pat. No. 568,037A; couplers represented by Formula (I) of lines 45 to 55 in Column 1 in US Pat. No. 5,066,576; couplers represented by Formula (I) in Paragraph 0008 in JP-A-4-274425; couplers of Claim 1 on page 40 in EP Pat. No. 498.381A1 (in particular, D-35 on page 18); couplers represented by Formula (Y) on page 4 in EP Pat. No. 447,969A1 (in particular, Y-1 (page 17) and Y-54 (page 41)); and couplers represented by Formulae (II) to (IV) on lines 36 to 58 of Column 7 in US Pat. No. 4,476,219 (in particular, II-17 and 19 (Column 17), and II-24 (Column 19)).

Magenta couplers: US Pat. Nos. 4,310,619 and 4,351,897, EP Pat. No. 73,636, US Pat. Nos. 3,061,432 and 3,725,067, Research Disclosure No. 24220 (June, 1984) and No. 24230 (June, 1984), JP-A-60-33552, JP-A-60-43659, JP-A-61-72238, JP-A-60-35730, JP-A-55-118034, JP-A-60-185951, US Pat. Nos. 4,500,630, 4,540,654, and 4,556,630, WO88/04795, JP-A-3-39737 (L-57 (page 11, lower right), L-68 (page 12, lower right), L-77 (page 13, lower right)), EP Pat. No. 456,257 [A-4]-63 (p. 134), [A-4]-73, -75 (p. 139), EP Pat. No. 486,965 M-4, -6 (p. 26), M-7 (p. 27), EP Pat. No. 571,959A M-45 (p. 19), JP-A-5-204106 M-1 (p. 6), and JP-A-4-362631 paragraph No. 0237, M-22, and US Pat. Nos. 3,061,432 and 3,725,067.

Cyan coupler: US Pat. Nos. 4,052,212, 4,146,396, 4,228,233, and 4,296,200, EP Pat. No. 73,636, JP-A-4-204843, CX-1, 3, 4, 5, 11, 12, 14, 15 (pp. 14 to 16); JP-A-4-43345 C-7, 10 (p. 35), 34, 35 (p. 37), (I-1), (I-17) (pp. 42 to 43); and couplers represented by Formulae (Ia) or (Ib) of Claim 1 in JP-A-6-67385.

In addition, couplers described in JP-A-62-215272 (page 91), JP-A-2-33144 (pages 3 and 30), and EP 355,660A (pages 4, 5, 45, and 47) are also useful.

Among the oil-soluble dyes represented by Formula (1) above, the magenta dyes particularly preferably used are dyes represented by Formula (3) below.

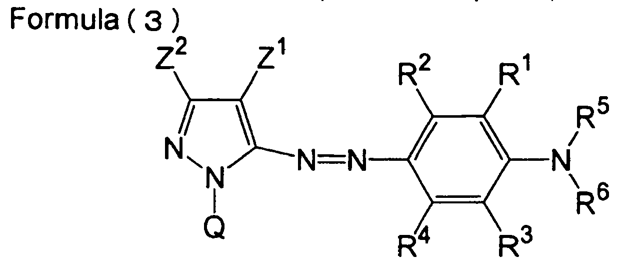

In Formula (3) above, Z¹ denotes an electron-attracting group having a Hammett substituent constant σp value of equal to or greater than 0.20. Z¹ is preferably an electron-attracting group having a σp value of at least 0.30 but no greater than 1.0. Preferred specific examples of the substituent include electron-attracting substituents that are described later, and among them an acyl group having 2 to 12 carbons, an alkyloxy carbonyl group having 2 to 12 carbons, a nitro group, a cyano group, an alkylsulfonyl group having 1 to 12 carbons, an arylsulfonyl group having 6 to 18 carbons, a carbamoyl group having 1 to 12 carbons, and a haloalkyl group having 1 to 12 carbons are preferable. A cyano group, an alkylsulfonyl group having 1 to 12 carbons, and an arylsulfonyl group having 6 to 18 carbons are particularly preferable, and a cyano group is most preferable.

In Formula (3) above, Z² denotes a hydrogen atom, an aliphatic group, or an aromatic group.

In Formula (3) above, R¹ to R⁶ are the same as the corresponding ones of Formula (1) above.

In Formula (3) above, Q denotes a hydrogen atom, an aliphatic group, an aromatic group, or a heterocyclic group. Among them, Q is preferably a group formed from a group of non-metal atoms necessary to form a 5- to 8-membered ring. Among them an aromatic group and a heterocyclic group are particularly preferable. The 5- to 8-membered ring may be substituted, may be a saturated ring, or may have an unsaturated bond. Preferred examples of the non-metal atom include a nitrogen atom, an oxygen atom, a sulfur atom, and a carbon atom. Specific examples of such ring structures include a benzene ring, a cyclopentane ring, a cyclohexane ring, a cycloheptane ring, a cyclooctane ring, a cyclohexene ring, a pyridine ring, a pyrimidine ring, a pyrazine ring, a pyridazine ring, a triazine ring, an imidazole ring, a benzoimidazole ring, an oxazole ring, a benzoxazole ring, an oxane ring, a sulfolane ring, and a thiane ring, and in a case where these rings have a further substituent, examples of the substituent include groups cited as examples of substituents R¹ to R⁴ in Formula (1) above.

Preferred structures of the compounds represented by Formula (3) above are described in JP-A-2001-335714.

Among the dyes represented by Formula (2) above, the magenta dye particularly preferably employs a dye represented by Formula (4) below.

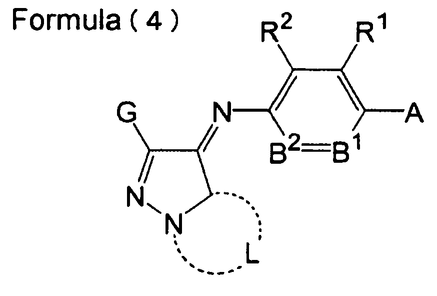

In Formula (4) above, G denotes a hydrogen atom, an aliphatic group, an aromatic group, a heterocyclic group, a cyano group, an alkoxy group, an aryloxy group, an alkylthio group, an arylthio group, an ester group, an amino group, a carbamoyl group, a sulfonyl group, a sulfamoyl group, a ureido group, a urethane group, an acyl group, an amide group, or a sulfonamide group.

In Formula (4) above, R¹, R², A, B¹, and B² are the same as the corresponding ones of Formula (2) above, and preferred ranges are also the same.

In Formula (4) above, L denotes an atomic group forming a five-membered or six-membered nitrogen-containing hetero ring, which may be substituted with at least one of an aliphatic group, an aromatic group, a heterocyclic group, a cyano group, an alkoxy group, an aryloxy group, an alkylthio group, an arylthio group, an ester group, an amino group, a carbamoyl group, a sulfonyl group, a sulfamoyl group, a ureido group, a urethane group, an acyl group, an amide group, and a sulfonamide group, and this hetero ring may further form a condensed ring with another ring.

With regard to compounds represented by Formula (4) above, A is preferably -NR⁵R⁶, and L preferably forms a five-membered nitrogen-containing hetero ring; examples of the five-membered nitrogen-containing hetero ring include an imidazole ring, a triazole ring, and a tetrazole ring.

Among the dyes represented by Formula (1) and Formula (2) above, compound examples (M-0 to 6, a-21 to 25) for a magenta dye are shown below, but these are only for explaining the present invention in detail, and the present invention should not be construed as being limited thereto.

In the present invention, M-0, M-4, M-6, or a-21 may be used, and M-4, M-6, and a-21 are particularly preferable.

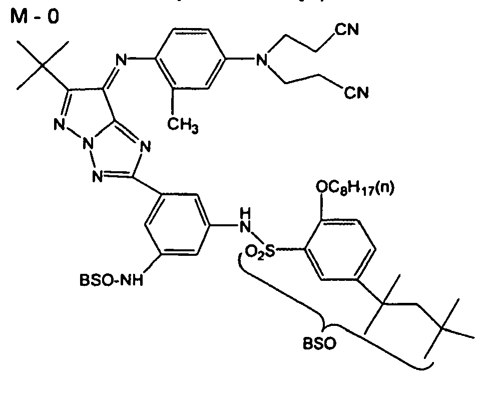

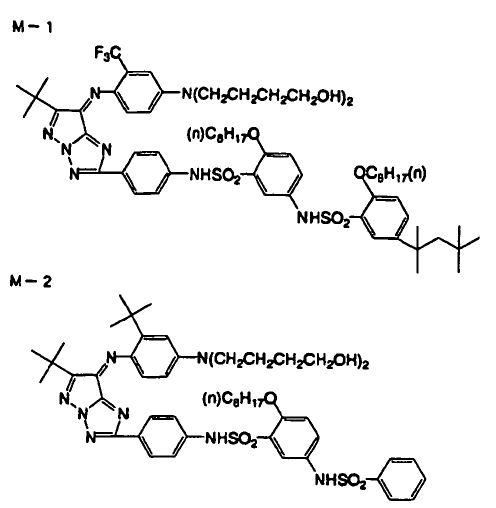

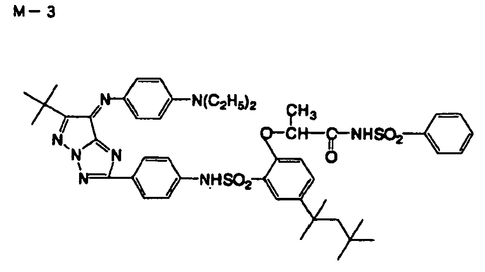

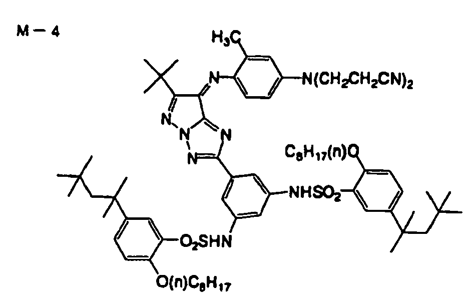

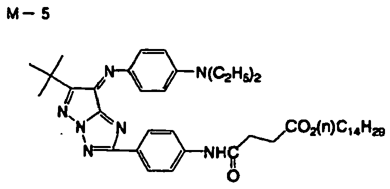

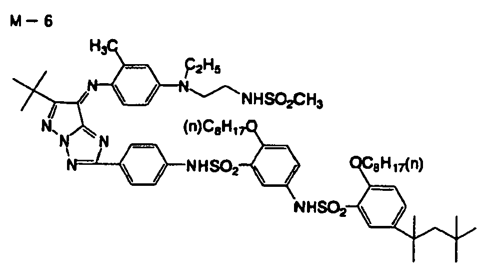

**(Table 1)**

| | | | | |
|---|---|---|---|---|
| 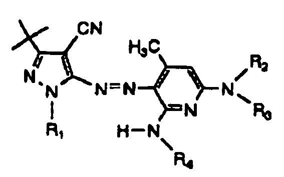 | | | | |

| Dye | R₁ | R₂ | R₃ | R₄ |
|---|---|---|---|---|
| a-21 | 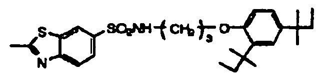 | 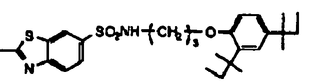 | 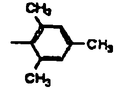 | 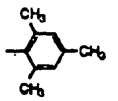 |
| a-22 | 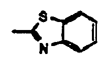 | 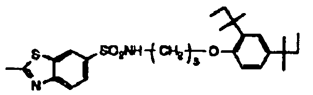 | 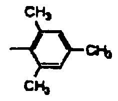 | 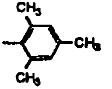 |
| a-23 | 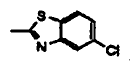 | 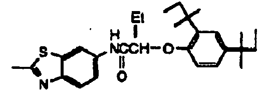 | 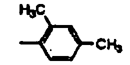 | 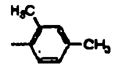 |
| a-24 | 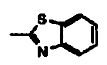 | 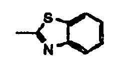 | 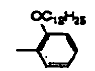 | 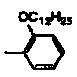 |
| a-25 | 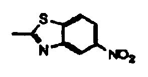 | 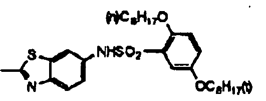 | 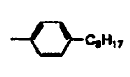 | C₈H₁₇(t) |

Other compound examples of the colorant that can be used in the present invention include those described in JP-A-2001-240763, 2001-181549, and JP-A-2001-335714, but the present invention should not be construed as being limited thereto.

The compound represented by Formula (3) above may be synthesized by reference to a method described in, for example, JP-A-2001-335714 or JP-A-55-161856. The compound represented by Formula (4) above may be synthesized by reference to a method described in, for example, JP-A-4-126772, JP-B-7-94180, or JP-A-2001-240763.

Among the dyes represented by Formula (2) above, as a cyan dye a pyrrolotriazole azomethine dye represented by Formula (5) below is particularly preferably used.

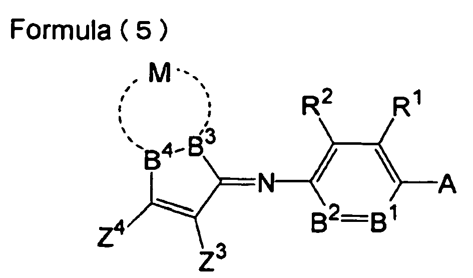

In Formula (5) above, A, R¹, R², B¹, and B² are the same as the corresponding ones of Formula (2) above, and preferred ranges thereof are also the same.

In Formula (5) above, Z³ and Z⁴ are independently the same as G in Formula (4) above. Z³ and Z⁴ may be bonded together to form a ring structure. One in which Z³ is an electron-attracting group having a Hammett substituent constant σp value of equal to or greater than 0.30 exhibits a sharp absorption and is more preferable. Moreover, one in which Z³ is an electron-attracting group having a Hammett substituent constant σp value of equal to or greater than 0.45 is more preferable, and an electron-attracting group having a Hammett substituent constant σp value of equal to or greater than 0.60 is most preferable. Furthermore, one in which the sum of the Hammett substituent constant σp values of Z³ and Z⁴ is equal to or greater than 0.70 exhibits excellent hue of a cyancolor, and is more preferable.

In Formula (5) above, M is an atomic group forming a 1,2,4-triazole ring that is condensed with the 5-membered ring of Formula (5) above; either one of the two atoms B³ and B⁴ at the sites of condensation with the 5-membered ring is a nitrogen atom, and the other is a carbon atom.

The compound represented by Formula (5) above is preferably used as a cyan dye, but it may be used as a magenta dye by changing a substituent.

The Hammett substituent constant σp value used in the present specification is now explained. The Hammett rule is an empirical rule proposed by L. P. Hammett in 1935 in order to quantitatively deal with the influence of a substituent on a reaction or equilibrium of a benzene derivative, and the validity thereof is currently widely recognized. A σp value and a σm value are required for the substituent constant in the Hammett rule, and details of these values can be referred to in many general books, for example, 'Lange's Handbook of Chemistry', Ed. by J. A. Dean, 12th edition, 1979 (Mc Graw-Hill) or 'Kagakuno Ryouiki' (Journal of Japanese Chemistry), special issue, 122, pp. 96 to 103, 1979 (Nankodo Co., Ltd.). In the present invention, the substituents are limited or explained using the Hammett substituent constant σp, but this does not mean that they are limited to substituents whose values are described in published references, and a substituent whose value is not published in the references but is included in the range if it is measured in accordance with the Hammett rule is of course included. Among Formulae (1) to (5) above, those that are not benzene derivatives are also included, but the σp value is used as a scale showing the electronic effect of the substituent, irrespective of the position of substitution. The σp value in the present invention is used with the above-mentioned meaning.

Examples of electron-attracting groups having a Hammett substituent constant σp value of equal to or greater than 0.60 include a cyano group, a nitro group, an alkylsulfonyl group (e.g. a methanesulfonyl group), and an arylsulfonyl group (e.g. a benzenesulfonyl group). Examples of electron-attracting groups having a Hammett σp value of equal to or greater than 0.45 include, in addition to the above, an acyl group (e.g. an acetyl group), an alkoxycarbonyl group (e.g. a dodecyloxycarbonyl group), an aryloxycarbonyl group (e.g. m-chlorophenoxycarbonyl), an alkylsulfinyl group (e.g. n-propylsulfinyl), an arylsulfinyl group (e.g. phenylsulfinyl), a sulfamoyl group (e.g. N-ethylsulfamoyl, N,N-dimethylsulfamoyl), and a haloalkyl group (e.g. trifluoromethyl).

Examples of electron-attracting groups having a Hammett substituent constant σp value of equal to or greater than 0.30 include, in addition to the above, an acyloxy group (e.g. acetoxy), a carbamoyl group (e.g. N-ethylcarbamoyl, N,N-dibutylcarbamoyl), a haloalkoxy group (e.g. trifluoromethyloxy), a haloaryloxy group (e.g. pentafluorophenyloxy), a sulfonyloxy group (e.g. a methylsulfonyloxy group), a haloalkylthio group (e.g. difluoromethylthio), an aryl group substituted with two or more electron-attracting groups having a σp value of equal to or greater than 0.15 (e.g. 2,4-dinitrophenyl, pentachlorophenyl), and a hetero ring (e.g. 2-benzooxazolyl, 2-benzothiazolyl, 1-phenyl-2-benzimidazolyl). Specific examples of electron-attracting groups having a σp value of equal to or greater than 0.20 include, in addition to the above, a halogen atom.

Furthermore, in the present invention, an oil-soluble dye represented by Formula (A-I) below can be used preferably.

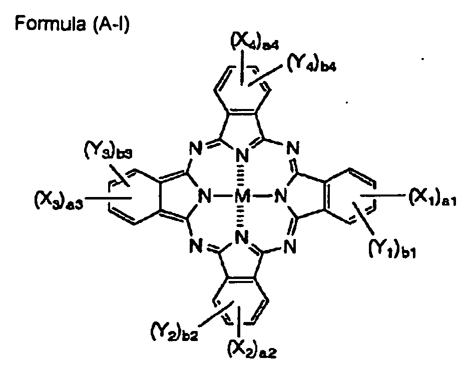

In Formula (A-I): X₁, X₂, X₃, and X₄ independently denote a group selected from -SO-Z, -SO₂-Z, -SO₂NR₁R₂, -CONR₁R₂, -CO₂R₁, and a sulfo group. Here, Z denotes a substituted or unsubstituted alkyl group, a substituted or unsubstituted cycloalkyl group, a substituted or unsubstituted alkenyl group, a substituted or unsubstituted aralkyl group, a substituted or unsubstituted aryl group, or a substituted or unsubstituted heterocyclic group. R₁ and R₂ independently denote a hydrogen atom, a substituted or unsubstituted alkyl group, a substituted or unsubstituted cycloalkyl group, a substituted or unsubstituted alkenyl group, a substituted or unsubstituted aralkyl group, a substituted or unsubstituted aryl group, or a substituted or unsubstituted heterocyclic group, provided that R₁ and R₂ are not both hydrogen atoms. M denotes a hydrogen atom, a metal element, a metal oxide, a metal hydroxide, or a metal halide. Y₁, Y₂, Y₃, and Y₄ independently denote a hydrogen atom or a monovalent substituent. a₁ to a₄ and b₁ to b₄ denote the numbers of X₁ to X₄ and Y₁ to Y₄, and independently denote an integer of 0 to 4, provided that the sum total of a₁ to a₄ is equal to or greater than 2.

Among the oil-soluble dyes represented by Formula (A-I) above, an oil-soluble dye represented by Formula (A-II) below may particularly preferably be used.

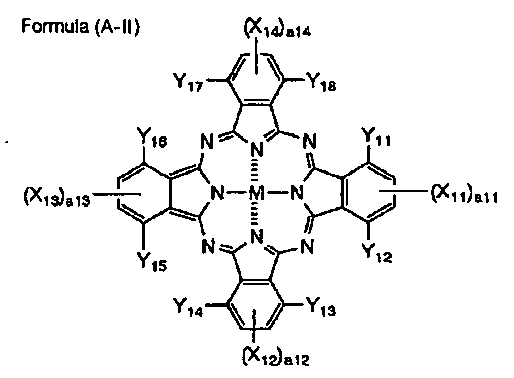

In Formula (A-II): X₁₁ to X₁₄, Y₁₁ to Y₁₈, and M are the same as X₁ to X₄, Y₁ to Y₄, and M in Formula (A-I) respectively. a₁₁ to a₁₄ independently denote an integer of 1 or 2.

As a specific example of Formula (A-II) above, a compound example (All-17 to 23) is cited, but this is for explaining the present invention in detail, and the present invention should not be construed as being limited thereto.

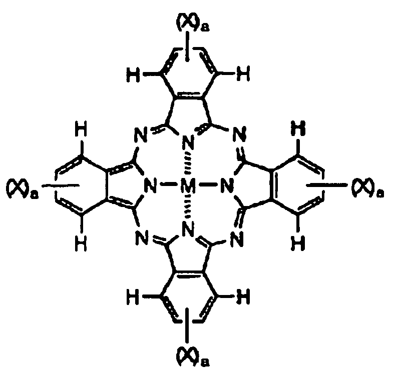

| Compound No. | M | X | a |
|---|---|---|---|
| AII-17 | Cu | 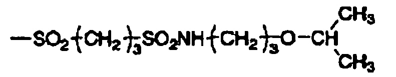 | 1 |
| AII-18 | Cu | 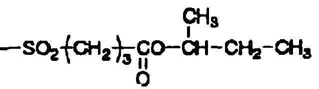 | 1 |
| AII-19 | Cu | 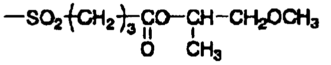 | 1 |
| AII-20 | Cu | 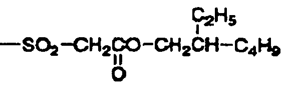 | 1 |
| AII-21 | Cu | 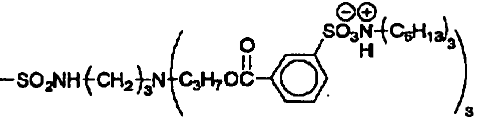 | 1 |
| AII-22 | Cu | 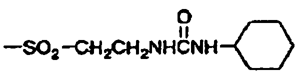 | 1 |
| AII-23 | Cu | 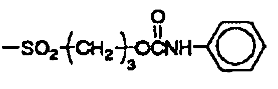 | 1 |

In the present invention, it is preferable to use an oil-soluble dye having an oxidation potential that is more noble than 1.0 V (SCE). The more noble the oxidation potential, the more preferable it is; it is more preferable to use one having an oxidation potential more noble than 1.1 V (SCE), and it is most preferable to use one having an oxidation potential more noble than 1.2 V (SCE).

The oxidation potential value (Eox) can be easily measured by one skilled in the art and a method therefor is described in, for example, P. Delahay, 'New Instrumental Methods in Electrochemistry', Interscience Publishers (1954), A. J. Bard et al., 'Electrochemical Methods', John Wiley & Sons (1980), and Akira Fujishima et al., 'Denkikagaku Sokuteihou' (Electrochemical Measurement Methods), Gihodo Shuppan Sha (1984).

More specifically, a test sample is dissolved to give a concentration of 1 x 10⁻⁴ to 1 x 10⁻⁶ mol/L in a solvent such as dimethylformamide or acetonitrile containing a supporting electrolyte such as sodium perchlorate or tetrapropylammonium perchlorate, an oxidation wave when sweeping toward the oxidation side (noble side) using carbon (GC) as a working electrode and a rotating platinum electrode as the counter electrode using cyclic voltammetry or direct current polarographic equipment is approximated to a straight line, and the oxidation potential of the midpoint of a line segment formed between an intersection point of the straight line and a residual current/potential straight line and an intersection point of the straight line and a saturated current straight line (or an intersection point with a straight line parallel to the ordinate passing through the potential peak value) is measured as a value relative to the SCE (saturated calomel electrode). This value sometimes deviates by on the order of tens of millivolts due to the effect of a liquid junction potential, the liquid resistance of the sample solution, or the like, but the reproducibility of the potential can be guaranteed by adding a standard sample (for example, hydroquinone). The support electrolyte and solvent used may be selected appropriately according to the oxidation potential and the solubility of the test sample. The support electrolyte and solvent that can be used here may be referred to in Akira Fujishima et al., 'Denkikagaku Sokuteihou' (Electrochemical Measurement Methods), Gihodo Shuppan Sha (1984), pp. 101 to 118.

In the concentration range of the above-mentioned measurement solvent and a phthalocyanine compound sample, the oxidation potential of a disassociated state is measured.

The value of Eox represents the ease of electron transfer from a sample to an electrode; the larger the value (the more noble the oxidation potential), the more difficult it is for electrons to transfer from the sample to the electrode, in other words, the more difficult it is to oxidize.

If a dye having a low oxidation potential is used, polymerization is greatly inhibited by the dye, and the curability is degraded. When a dye having a noble oxidation potential is used, there is hardly any inhibition of polymerization.

The coloring agent that can be used in the present invention is preferably added to the ink composition or the inkjet recording ink composition of the present invention and then dispersed in the ink to an appropriate degree. For dispersion of the coloring agent, for example, a dispersing machine such as a ball mill, a sand mill, an attritor, a roll mill, an agitator, a Henschel mixer, a colloidal mill, an ultrasonic homogenizer, a pearl mill, a wet type jet mill, or a paint shaker may be used.

When carrying out dispersion of the coloring agent, a dispersant may be added. The type of dispersant is not particularly limited, but it is preferable to use a polymeric dispersant, and examples of the polymeric dispersant include the Solsperse series manufactured by Noveon. Furthermore, as a dispersion adjuvant, it is also possible to use a synergist, according to the various types of pigment. In the present invention, the dispersant and dispersion adjuvant are preferably added at 1 to 50 parts by weight relative to 100 parts by weight of the pigment.

The coloring agent may be added directly to the ink composition of the present invention, but in order to improve dispersibility it may be added in advance to a solvent or a dispersing medium such as a polymerizable compound used in the present invention. In the present invention, in order to avoid the problem of the solvent resistance being degraded when the solvent remains in the cured image and the VOC (Volatile Organic Compound) problem of the residual solvent, it is preferable to add the coloring agent to a polymerizable compound. As a polymerizable compound used, it is preferable in terms of dispersion suitability to select a monomer having the lowest viscosity.

In the present invention, it is preferable for the average particle size of the coloring agent to be in the range of 0.005 to 0.5 µm, more preferably 0.01 to 0.45 µm, and yet more preferably, 0.015 to 0.3 µm. Furthermore, it is preferable for the maximum particle size of the coloring agent to be 0.3 to 10 µm, and more preferably 0.3 to 3 µm. It is preferable, in order to make the maximum particle size of the pigment particles be in the above-mentioned range, that the coloring agent, the dispersant, and the dispersing medium are selected, and dispersion conditions and filtration conditions are set. By such control of particle size, clogging of a head nozzle can be suppressed, and the ink storage stability, the ink transparency, and the curing sensitivity can be maintained, which is preferable.

When the colorant is used, the content of the colorant is preferably 1 to 50 wt % of the weight of the entire ink, and more preferably 2 to 30 wt %.

### (e) Cosensitizer

The ink composition of the present invention preferably comprises a cosensitizer. In the present invention, the cosensitizer has the function of further improving the sensitivity of the sensitizer to actinic radiation or the function of suppressing inhibition by oxygen of polymerization of a polymerizable compound, etc.

Examples of such a cosensitizer include amines such as compounds described in M. R. Sander et al., 'Journal of Polymer Society', Vol. 10, p. 3173 (1972), JP-B-44-20189, JP-A-51-82102, JP-A-52-134692, JP-A-59-138205, JP-A-60-84305, JP-A-62-18537, JP-A-64-33104, and Research Disclosure No. 33825, and specific examples thereof include triethanolamine, ethyl p-dimethylaminobenzoate, p-formyldimethylaniline, and p-methylthiodimethylaniline.

Other examples of the cosensitizer include thiols and sulfides such as thiol compounds described in JP-A-53-702, JP-B-55-500806, and JP-A-5-142772, and disulfide compounds of JP-A-56-75643, and specific examples thereof include 2-mercaptobenzothiazole, 2-mercaptobenzoxazole, 2-mercaptobenzimidazole, 2-mercapto-4(3H)-quinazoline, and β-mercaptonaphthalene.

Yet other examples of the cosensitizer include amino acid compounds (e.g. N-phenylglycine, etc.), organometallic compounds described in JP-B-48-42965 (e.g. tributyltin acetate, etc.), hydrogen-donating compounds described in JP-B-55-34414, sulfur compounds described in JP-A-6-308727 (e.g. trithiane, etc.), and phosphorus compounds described in JP-A-6-250387 (diethylphosphite, etc.).

### (f) Other polymerization initiator

In the present invention, the ink composition may employ in combination (a) a sulfonium salt having a cation represented by Formula (II) (sulfonium salt (A)) and (f) another polymerization initiator. As a polymerization initiator used in combination, a known radical polymerization initiator can be cited as an example. Said other polymerization initiator may be used singly or in a combination of two or more types.

In the present invention, the radical polymerization initiator that can be used in the ink composition is a compound that generates a polymerization initiating species by absorbing external energy. The external energy that is used for initiating polymerization can be broadly divided into heat and actinic radiation, and a thermopolymerization initiator and a photopolymerization initiator are used respectively. Examples of the actinic radiation include γ rays, β rays, an electron beam, UV rays, visible light, and IR rays. The polymerization initiator that can be used in the present invention is preferably a radiation-sensitive radical polymerization initiator, which is sensitive to actinic radiation, a so-called radical photopolymerization initiator.

Preferred examples of the radical polymerization initiator that can be used in combination in the present invention include (a) an aromatic ketone, (b) an organic peroxide, (c) a thio compound, (d) a hexaarylbiimidazole compound, (e) a ketoxime ester compound, (f) a borate compound, (g) an azinium compound, (h) a metallocene compound, (i) an active ester compound, (j) a compound having a carbon-halogen bond, and (k) an alkylamine compound. With regard to these radical polymerization initiators, the above-mentioned compounds (a) to (k) may be used singly or in combination.

### (g) Other components

The ink composition of the present invention may comprise other components as necessary. Examples of the other components include basic compounds, polymerization inhibitors, and solvents.

It is preferable to add the basic compound from the viewpoint of improving the storage stability of the ink composition. As the basic compound that can be used in the present invention, a known basic compound may be used and, for example, a basic inorganic compound such as an inorganic salt or a basic organic compound such as an amine is preferably used.

It is preferable to add the polymerization inhibitor from the viewpoint of enhancing the storage stability. When the ink composition of the present invention is used as an inkjet recording ink composition, it is preferably heated in the range of 40°C to 80°C to thus make it less viscous and then discharged, and in order to prevent clogging of a head due to thermal polymerization it is preferable to add a polymerization inhibitor. The polymerization inhibitor is preferably added at 200 to 20,000 ppm relative to the total amount of the ink composition of the present invention. Examples of the polymerization inhibitor include hydroquinone, benzoquinone, p-methoxyphenol, TEMPO, TEMPOL, and Al cupferron.

While taking into consideration the ink composition and the inkjet recording ink composition of the present invention being radiation curing type ink compositions, it is preferable for them not to contain any solvent so that the ink compositions can react quickly and be cured immediately after landing. However, as long as the curing speed, etc. of the ink composition is not affected, a specified solvent may be added. In the present invention, as a solvent, an organic solvent or water may be used. In particular, the organic solvent may be added in order to improve the adhesion to a recording medium (a support such as paper). Adding an organic solvent having a high boiling point is effective since the problem with VOC can be avoided. The amount of organic solvent is preferably 0.1 to 5 wt % relative to the total amount of the ink composition of the present invention, and more preferably 0.1 to 3 wt %.

As means for preventing the sensitivity from being degraded by a light blocking effect of the coloring agent, which may be added to the ink composition, a combination of a cationically polymerizable compound and a cationic polymerization initiator, a combination of a radically polymerizable compound and a radical polymerization initiator, or a radical/cationic hybrid curing ink combining a polymerizable compound and a polymerization initiator may be employed.

In addition to the above, the ink composition of the present invention may contain a known compound as necessary. Examples thereof include a surfactant, a leveling additive, a matting agent and, for adjusting film physical properties, a polyester resin, polyurethane resin, vinyl resin, acrylic resin, rubber resin, or wax, which may be appropriately selected and added. Furthermore, in order to improve the adhesion to a recording medium such as a polyolefin or PET, a tackifier that does not inhibit polymerization is preferably added. Specific examples of the tackifier include high molecular weight tacky polymers described on pp. 5 and 6 of JP-A-2001-49200 (e.g. a copolymer formed from an ester of (meth)acrylic acid and an alcohol having an alkyl group with 1 to 20 carbons, an ester of (meth)acrylic acid and an alicyclic alcohol having 3 to 14 carbons, or an ester of (meth)acrylic acid and an aromatic alcohol having 6 to 14 carbons), and a low molecular weight tackifying resin having a polymerizable unsaturated bond.

When the ink composition thus obtained is used for inkjet recording, while taking into consideration dischargability, the viscosity of the ink composition at the discharge temperature (e.g. preferably 25°C to 80°C, more preferably 30°C to 75°C) is preferably 7 to 30 mPa·s, and more preferably 7 to 20 mPa·s. For example, the ink composition of the present invention has a viscosity at room temperature (25°C to 30°C) of preferably 35 to 500 mPa·s, and more preferably 35 to 200 mPa·s. With regard to the ink composition of the present invention, it is preferable that its component ratio is appropriately adjusted so that the viscosity is in the above-mentioned range. When the viscosity at room temperature is set to be high, even when a porous recording medium is used, penetration of the ink into the recording medium can be prevented, uncured monomer can be reduced, and the odor can be reduced. Furthermore, ink spreading when ink droplets have landed can be suppressed, and as a result there is the advantage that the image quality is improved.

As the recording medium to which the ink composition of the present invention can be applied, papers such as ordinary uncoated paper and coated paper, various types of non-absorbent resin material used as so-called flexible packaging, and resin films formed by molding the material into a film may be used, and examples of various types of plastic films include polyethylene terephthalate (PET) film, oriented polystyrene (OPS) film, oriented polypropyrene (OPP) film, oriented nyron (ONy) film, polyviylchrolide (PVC) film, polyethylene (PE) film, and triacetylcellulose (TAC) film. Other examples of plastic that can be used as a recording medium material include polycarbonate, acrylic resin, ABS, polyacetal, PVA, and rubber. Furthermore, metal and glass may be used as a recording medium.

The surface energy of these various types of plastic films differs depending on the properties of the material, and variation in the dot diameter after the ink lands has been a problem for some recording materials. In accordance with the configuration of the present invention, a good high precision image can be formed on recording materials having a wide range of surface energies from 35 to 60 mN/m, including an OPP film and an OPS film, which have a low surface energy, and PET, which has a relatively high surface energy.

### (3) Inkjet recording method and inkjet recording device

The ink composition of the present invention is preferably for inkjet recording use.

An inkjet recording method and an inkjet recording device that are desirably employed in the present invention are now explained.

### (3-1) Inkjet recording method

The present invention provides a method for forming an image by discharging the above-mentioned ink composition onto a recording medium (support, recording material, etc.) and curing the ink composition by irradiating the ink composition so discharged onto the recording medium with actinic radiation. That is, the present invention relates to an inkjet recording method comprising:
(a) a step of discharging an ink composition onto a recording medium; and
(b) a step of curing the ink composition by irradiating the ink composition so discharged with actinic radiation,
wherein the ink composition is the ink composition of the present invention.

The cured ink composition forms an image on the recording medium.

The peak wavelength of the actinic radiation is preferably 200 to 600 nm, more preferably 300 to 450 nm, and yet more preferably 350 to 420 nm. The output of the actinic radiation is preferably no greater than 2,000 mJ/cm², and is more preferably 10 to 2,000 mJ/cm², yet more preferably 20 to 1,000 mJ/cm², and particularly preferably 50 to 800 mJ/cm².

The inkjet recording method of the present invention is explained by taking as an example a process for producing a lithographic printing plate, the process comprising discharging an ink composition onto the lithographic printing plate so as to form an image.

A process for producing a lithographic printing plate of the present invention comprises:
(a) a step of discharging the ink composition of the present invention onto a hydrophilic support, and
(b) a step of irradiating the discharged ink composition with radiation so as to cure the ink composition, thus forming a hydrophobic image on the hydrophilic support by curing the ink composition.

### (3-1-1) Hydrophilic support used for lithographic printing plate

The lithographic printing plate comprises a support and an image formed on the support.

Conventionally, as the lithographic printing plate, a so-called PS plate in which an oleophilic photosensitive resin layer is provided on a hydrophilic support has been widely used. In a process for producing this PS plate, normally, after a mask exposure (surface exposure) is carried out via a lith film, non-exposed areas are dissolved and removed to give a desired printing plate. However, in recent years, a technique of digitizing image information using a computer by electronically processing, storing, and outputting the information has become widespread, and a new image output system that can be used for the above technique has been desired. In particular, a computer to plate (CTP) technique in which a printing plate is directly produced by scanning according to digitized image information with highly coherent light such as laser light without using a lith film has been developed.

As a system for obtaining a lithographic printing plate that makes possible the above scanning exposure, a process for directly producing a lithographic printing plate using an ink composition or an inkjet recording ink composition can be cited. This process involves obtaining a printing plate having a desired image (preferably a hydrophobic image) by discharging an ink onto a support, and preferably a hydrophilic support, using an inkjet system, etc., and exposing this to actinic radiation so as to expose an area with the ink composition or the inkjet recording ink to light. The ink composition or the inkjet recording ink suitable for such a system is the ink composition or the inkjet recording ink of the present invention.

The support (recording medium) onto which the ink composition or the inkjet recording ink composition of the present invention is discharged is not particularly limited, and a dimensionally stable sheet-form support may be used. The support is preferably a hydrophilic support. Examples of materials forming the support include paper, paper laminated with a plastic (e.g. polyethylene, polypropylene, polystyrene, etc.), a metal sheet (e.g. aluminum, zinc, copper, etc.), a plastic film (e.g. cellulose diacetate, cellulose triacetate, cellulose propionate, cellulose butyrate, cellulose acetate butyrate, cellulose nitrate, polyethylene terephthalate, polyethylene, polystyrene, polypropylene, polycarbonate, polyvinyl acetal, etc.), and paper or plastic film on which the above-mentioned metal is laminated or vapor-deposited. Preferred examples of the support include a polyester film and aluminum sheet. Among these, aluminum sheet is particularly preferable since the dimensional stability is good and it is relatively inexpensive.

The aluminum sheet is a pure aluminum sheet, an alloy sheet containing aluminum as a main component and a small amount of a different element, or a thin film of aluminum or an aluminum alloy laminated with a plastic. Examples of the different element contained in the aluminum alloy include silicon, iron, manganese, copper, magnesium, chromium, zinc, bismuth, nickel, and titanium. The content of the different element in the alloy is preferably equal to or less than 10 wt %. In the present invention, a pure aluminum sheet is preferable, but since it is difficult to produce completely pure aluminum because of the refining technique, a trace amount of a different element may be contained. The composition of the aluminum sheet is not specified, and a known generally used material may be utilized as appropriate.

The support preferably has a thickness of 0.1 to 0.6 mm, and more preferably 0.15 to 0.4 mm.

Prior to the aluminum sheet being used, it is preferably subjected to a surface treatment such as a surface roughening treatment or an anodizing treatment. Surface treatment makes it easy to improve the hydrophilicity and ensure that there is good adhesion between an image recording layer and the support. Prior to the aluminum sheet being subjected to the surface roughening treatment, it may be subjected as desired to a degreasing treatment using a surfactant, an organic solvent, an aqueous alkaline solution, etc. in order to remove rolling oil on the surface.

The surface roughening treatment for the aluminum sheet surface may be carried out by various types of methods, and examples thereof include a mechanical surface roughening treatment, an electrochemical surface roughening treatment (a surface roughening treatment involving dissolving the surface electrochemically), and a chemical surface roughening treatment (a surface roughening treatment involving selectively dissolving the surface chemically).

As a method for the mechanical surface roughening treatment, a known method such as a ball grinding method, a brush grinding method, a blast grinding method, or a buff grinding method may be used. It is also possible to use a transfer method in which an irregular shape is transferred using a roller provided with irregularities in an aluminum rolling stage.

As a method for the electrochemical surface roughening treatment, for example, a method in which alternating current or direct current is applied in an electrolyte solution containing an acid such as hydrochloric acid or nitric acid can be cited. It is also possible to employ a method as described in JP-A-54-63902 in which a mixed acid is used.

The aluminum sheet subjected to a surface roughening treatment is subjected as necessary to an alkali etching treatment using an aqueous solution of potassium hydroxide, sodium hydroxide, etc.; furthermore, after neutralization, it may be subjected to an anodizing treatment as desired in order to improve the abrasion resistance.

As an electrolyte that may be used for the anodizing treatment of the aluminum sheet, various types of electrolytes that form a porous oxide film may be used. In general, sulfuric acid, hydrochloric acid, oxalic acid, chromic acid, or a mixed acid thereof may be used. The concentration of the electrolyte may be determined as appropriate according to the type of electrolyte.

Conditions for the anodizing treatment depend on the type of electrolyte used and cannot be specified, but in general the electrolyte solution concentration is 1 to 80 wt %, the solution temperature is 5°C to 70°C, the current density is 5 to 60 A/dm², the voltage is 1 to 100V, and the electrolysis time is 10 sec. to 5 min. The amount of anodized film formed is preferably 1.0 to 5.0 g/m², and more preferably 1.5 to 4.0 g/m². It is preferable for it to be in this range since good plate life and good scratch resistance of a non-image area of a lithographic printing plate can be obtained.

As the support that can be used in the present invention, a substrate that has been subjected to the above-mentioned surface treatment and has an anodized film may be used as it is, but in order to further improve the adhesion to an upper layer, and the hydrophilicity, the contamination resistance, the thermal insulation, etc., the substrate may appropriately be subjected as necessary to a treatment for enlarging micropores of the anodized film, a sealing treatment, or a surface hydrophilization treatment involving immersion in an aqueous solution containing a hydrophilic compound, which are described in JP-A-2001-253181 or JP-A-2001-322365. These enlarging and sealing treatments are not limited to those described therein, and any conventionally known methods may be employed.

### Sealing treatment

The sealing treatment may be vapor sealing, a treatment with an aqueous solution containing an inorganic fluorine compound such as a single treatment with fluorozirconic acid or a treatment with sodium fluoride, vapor sealing with added lithium chloride, or a sealing treatment with hot water.

Among these, the sealing treatment with an aqueous solution containing an inorganic fluorine compound, the sealing treatment with vapor, and the sealing treatment with hot water are preferable. Each thereof is explained below.

### Sealing treatment with aqueous solution containing inorganic fluorine compound

In the sealing treatment with an aqueous solution containing an inorganic fluorine compound, a metal fluoride can suitably be used as the inorganic fluorine compound.

Specific examples thereof include sodium fluoride, potassium fluoride, calcium fluoride, magnesium fluoride, sodium fluorozirconate, potassium fluorozirconate, sodium fluorotitanate, potassium fluorotitanate, ammonium fluorozirconate, ammonium fluorotitanate, potassium fluorotitanate, fluorozirconic acid, fluorotitanic acid, hexafluorosilicic acid, nickel fluoride, iron fluoride, fluorophosphoric acid, and ammonium fluorophosphate. Among them, sodium fluorozirconate, sodium fluorotitanate, fluorozirconic acid, and fluorotitanic acid are preferable.

The concentration of the inorganic fluorine compound in the aqueous solution is preferably at least 0.01 wt % from the viewpoint of sealing of micropores on an anodized coating being carried out sufficiently, and more preferably at least 0.05 wt %, and it is preferably no greater than 1 wt % from the viewpoint of contamination resistance, and more preferably no greater than 0.5 wt %.

The aqueous solution containing an inorganic fluorine compound preferably further contains a phosphate compound. It is preferable for a phosphate compound to be contained since the hydrophilicity of the surface of the anodized coating improves and the machine developability and the contamination resistance can be improved.

Preferred examples of the phosphate compound include phosphates of a metal such as an alkali metal or an alkaline earth metal.

Specific examples thereof include zinc phosphate, aluminum phosphate, ammonium phosphate, ammonium phosphate dibasic, ammonium dihydrogen phosphate, potassium dihydrogen phosphate, potassium phosphate dibasic, calcium phosphate, ammonium sodium hydrogen phosphate, magnesium hydrogen phosphate, magnesium phosphate, ferrous phosphate, ferric phosphate, sodium dihydrogen phosphate, sodium phosphate, sodium phosphate dibasic, lead phosphate, diammonium phosphate, calcium dihydrogen phosphate, lithium phosphate, phosphotungstic acid, ammonium phosphotungstate, sodium phosphotungstate, ammonium phosphomolybdate, sodium phosphomolybdate, sodium phosphite, sodium tripolyphosphate, and sodium pyrophosphate. Among these, sodium dihydrogen phosphate, sodium phosphate dibasic, potassium dihydrogen phosphate, and potassium phosphate dibasic are preferable.

The combination of the inorganic fluorine compound and the phosphate compound is not particularly limited, but the aqueous solution preferably comprises at least sodium fluorozirconate as the inorganic fluorine compound and at least sodium dihydrogen phosphate as the phosphate compound.

The concentration of the phosphate compound in the aqueous solution is preferably at least 0.01 wt % from the viewpoint of improving machine developability and contamination resistance, and more preferably at least 0.1 wt %, and it is preferably no greater than 20 wt % from the viewpoint of solubility, and more preferably no greater than 5 wt %.

The proportion of each compound in the aqueous solution is not particularly limited, but the ratio by weight of the inorganic fluorine compound and the phosphate compound is preferably 1/200 to 10/1, and more preferably 1/30 to 2/1.

Furthermore, the temperature of the aqueous solution is preferably at least 20°C, and more preferably at least 40°C, and it is preferably no higher than 100°C, and more preferably no higher than 80°C.

Moreover, the pH of the aqueous solution is preferably at least 1, and more preferably at least 2, and it is preferably no greater than 11, and more preferably no greater than 5.

A method for the sealing treatment with the aqueous solution containing an inorganic fluorine compound is not particularly limited and, for example, an immersion method and a spray method may be used. They may be employed once or a plurality of times, or in a combination of two or more types.

Among these, the immersion method is preferable. When the treatment is carried out by the immersion method, the treatment time is preferably at least 1 sec., and more preferably at least 3 sec., and it is preferably no greater than 100 sec., and more preferably no greater than 20 sec.

### Sealing treatment with steam

With regard to the sealing treatment with steam, for example, a method in which an anodized coating is contacted with steam at high pressure or normal pressure continuously or discontinuously can be cited.

The temperature of the steam is preferably at least 80°C, and more preferably at least 95°C, and it is preferably no greater than 105°C.

The pressure of the steam is preferably in the range of (atmospheric pressure - 50 mmAq) to (atmospheric pressure + 300 mmAq) (1.008 x 10⁵ to 1.043 x 10⁵ Pa).

Furthermore, the time for which the coating is contacted with steam is preferably at least 1 sec., and more preferably at least 3 sec., and it is preferably no greater than 100 sec., and more preferably no greater than 20 sec.

### Sealing treatment with hot water

With regard to the sealing treatment with hot water, for example, a method in which an aluminum plate having an anodized coating formed thereon is immersed in hot water can be cited.

The hot water may contain an inorganic salt (e.g. a phosphate) or an organic salt.

The temperature of the hot water is preferably at least 80°C, and more preferably at least 95°C, and it is preferably no greater than 100°C.

Furthermore, the time for which immersion in hot water is carried out is preferably at least 1 sec., and more preferably at least 3 sec., and it is preferably no greater than 100 sec., and more preferably no greater than 20 sec.

With regard to a hydrophilization treatment that is used in the present invention, there is an alkali metal silicate method, as disclosed in US Pat. Nos. 2,714,066, 3,181,461, 3,280,734, and 3,902,734. In this method, a support is immersed in an aqueous solution of sodium silicate, etc., or subjected to electrolysis. In addition, there is a method in which a support is treated with potassium fluorozirconate, as described in JP-B-36-22063, and a method in which a support is treated with polyvinylphosphonic acid, as described in UP Pat. Nos. 3,276,868, 4,153,461, and 4,689,272.

In the present invention, it is preferable for the support to have a center line average roughness of 0.10 to 1.2 µm. It is preferable for it to be in this range since good adhesion to an image recording layer, good plate life, and good contamination resistance can be obtained.

### (3-1-2) Step of discharging ink composition onto hydrophilic support

When the ink composition or the inkjet recording ink composition of the present invention is discharged onto the surface of the above-mentioned hydrophilic support, the ink composition or the inkjet recording ink composition is preferably discharged after being heated to preferably 25°C to 80°C, and more preferably 30°C to 75°C, so as to reduce the viscosity of the ink composition to preferably 5 to 30 mPa·s, and more preferably 7 to 20 mPa·s. In particular, it is preferable to use the ink composition having an ink viscosity at 25°C of 35 to 500 mPa·s since a large effect can be obtained. By employing this method, high discharge stability can be realized. The radiation curing type ink composition such as the ink composition of the present invention generally has a viscosity that is higher than that of a normal ink composition or a water-based ink used for an inkjet recording ink, and variation in viscosity due to a change in temperature at the time of discharge is large. Viscosity variation in the ink has a large effect on changes in liquid droplet size and changes in liquid droplet discharge speed and, consequently, causes the image quality to be degraded. It is therefore necessary to maintain the ink discharge temperature as constant as possible. In the present invention, the control range for the temperature is desirably ±5°C of a set temperature, preferably ±2°C of the set temperature, and more preferably ±1°C of the set temperature.

### (3-1-3) Step of curing ink composition by irradiating discharged ink composition with actinic radiation so as to form hydrophobic image on hydrophilic support by curing ink composition

The ink composition discharged onto the surface of the hydrophilic support is cured by irradiating with actinic radiation. This results from a sensitizing dye in a polymerization initiation system contained in the above-mentioned ink composition of the present invention absorbing actinic radiation, attaining an excited state, and coming into contact with a polymerization initiator in the polymerization initiation system to thus decompose the polymerization initiator, and a polymerizable compound undergoing radical polymerization and being cured.

The actinic radiation used in this process may include α rays, γ rays, an electron beam, X rays, UV rays, visible light, and IR rays. Although it depends on the absorption characteristics of the sensitizing dye, the peak wavelength of the actinic radiation is, for example, 200 to 600 nm, preferably 300 to 450 nm, and more preferably 350 to 450 nm. Furthermore, in the present invention, the polymerization initiation system has sufficient sensitivity for low output actinic radiation. The output of the actinic radiation as irradiation energy is therefore, for example, 2,000 mJ/cm² or less, and is preferably 10 to 2,000 mJ/cm², more preferably 20 to 1,000 mJ/cm², and yet more preferably 50 to 800 mJ/cm². Moreover, the actinic radiation is applied so that the illumination intensity on the exposed surface is, for example, 10 to 2,000 mW/cm², and preferably 20 to 1,000 mW/cm².

The ink composition of the present invention is desirably exposed to such actinic radiation for, for example, 0.01 to 120 sec., and preferably 0.1 to 90 sec.

Irradiation conditions and a basic method for irradiation with actinic radiation are disclosed in JP-A-60-132767. Specifically, a light source is provided on either side of a head unit that includes an ink discharge device, and the head unit and the light source are made to scan by a so-called shuttle system. Irradiation with actinic radiation is carried out after a certain time (e.g. 0.01 to 0.5 sec., preferably 0.01 to 0.3 sec., and more preferably 0.01 to 0.15 sec.) has elapsed from when the ink has landed. By controlling the time from ink landing to irradiation so as to be a minimum in this way, it becomes possible to prevent the ink that has landed on a recording medium from spreading before being cured. Furthermore, since the ink can be exposed before it reaches a deep area of a porous recording medium that the light source cannot reach, it is possible to prevent monomer from remaining unreacted, and as a result the odor can be reduced.

Furthermore, curing may be completed using another light source that is not driven. WO99/54415 discloses, as an irradiation method, a method employing an optical fiber and a method in which a collimated light source is incident on a mirror surface provided on a head unit side face, and a recorded area is irradiated with UV light.

By employing such a recording method, it is possible to maintain a uniform dot diameter for landed ink even for various types of recording media having different surface wettability, thereby improving the image quality. In order to obtain a color image, it is preferable to superimpose colors in order from those with a low lightness. By superimposing inks in order from one with low lightness, it is easy for radiation to reach a lower ink, the curing sensitivity is good, the amount of residual monomer decreases, odor is reduced, and an improvement in adhesion can be expected. Furthermore, although it is possible to discharge all colors and then expose them at the same time, it is preferable to expose one color at a time from the viewpoint of promoting curing.

In this way, the above-mentioned ink composition of the present invention is cured by irradiation with actinic radiation to thus form a hydrophobic image on the surface of the hydrophilic support.

### (3-2) Inkjet recording device

The inkjet recording device used in the present invention is not particularly restricted, and a commercial inkjet recording device may be used. That is, in the present invention, recording on a recording medium may be carried out using a commercial inkjet recording device.

The inkjet recording device that can be used in the present invention is equipped with, for example, an ink supply system, a temperature sensor, and an actinic radiation source.

The ink supply comprises, for example, a main tank containing the ink composition of the present invention, a supply pipe, an ink supply tank immediately before an inkjet head, a filter, and a piezo system inkjet head. The piezo system inkjet head may be driven so as to discharge a multisize dot of 1 to 100 pL, and preferably 8 to 30 pL, at a resolution of 320 x 320 to 4,000 x 4,000dpi, preferably 400 x 400 to 1,600 x 1,600dpi, and more preferably 720 x 720 dpi. Here, dpi referred to in the present invention means the number of dots per 2.54 cm.

As described above, since it is desirable for the radiation curing type ink to be discharged at a constant temperature, a section from the ink supply tank to the inkjet head is thermally insulated and heated. A method of controlling temperature is not particularly limited, but it is preferable to provide, for example, temperature sensors at a plurality of pipe section positions, and control heating according to the ink flow rate and the temperature of the surroundings. The temperature sensors may be provided on the ink supply tank and in the vicinity of the inkjet head nozzle. Furthermore, the head unit that is to be heated is preferably thermally shielded or insulated so that the device main body is not influenced by the temperature of the outside air. In order to reduce the printer start-up time required for heating, or in order to reduce the thermal energy loss, it is preferable to thermally insulate the head unit from other sections and also to reduce the heat capacity of the entire heated unit.

As an actinic radiation source, a mercury lamp, a gas/solid laser, etc. are mainly used, and for UV photocuring inkjet a mercury lamp and a metal halide lamp are widely known. However, from the viewpoint of protection of the environment, there has recently been a strong desire for mercury not to be used, and replacement by a GaN semiconductor UV light emitting device is very useful from industrial and environmental viewpoints. Furthermore, LEDs (UV-LED) and LDs (UV-LD) have small dimensions, long life, high efficiency, and low cost, and their use as a photocuring inkjet light source can be expected.

Furthermore, light-emitting diodes (LED) and laser diodes (LD) may be used as the source of actinic radiation. In particular, when a UV ray source is needed, a UV-LED or a UV-LD may be used. For example, Nichia Corporation has marketed a violet LED having a wavelength of the main emission spectrum of between 365 nm and 420 nm. Furthermore, when a shorter wavelength is needed, US Pat. No. 6,084,250 discloses an LED that can emit actinic radiation whose wavelength is centered between 300 nm and 370 nm. Furthermore, another violet LED is available, and irradiation can be carried out with radiation of a different UV bandwidth. The actinic radiation source particularly preferable in the present invention is a UV-LED, and a UV-LED having a peak wavelength at 350 to 420 nm is particularly preferable.

The maximum illumination intensity of the LED on a recording medium is preferably 10 to 2,000 mW/cm², more preferably 20 to 1,000 mW/cm², and particularly preferably 50 to 800 mJ/cm².

### Examples

The present invention is explained more specifically below by reference to Examples and Comparative Examples, but the present invention should not be construed as being limited to the modes of these Examples.

### Synthetic example of sulfonium salt having cation represented by Formula (II)

### Synthesis of Sulfonium salt A (Comparative Example)

3.0 g of dibenzothiophene oxide was added to 50 mL of toluene and stirred. The mixture was maintained at 0°C, 6.3 g of trifluoroacetic anhydride was slowly added dropwise thereto, stirring was carried out at 0°C for 30 minutes, 4.5 g of nonafluorobutanesulfonic acid was then slowly added dropwise, stirring was carried out at 0°C for 3 hours, following this the mixture was poured into 100 mL of water, which was extracted with 50 mL of toluene, and the aqueous phase was further extracted with 50 mL of toluene. The organic phase was washed once with saturated brine. The organic phase was concentrated, 40 mL of methanol was added thereto, and the mixture was slowly added dropwise to 90 mL of a 0.5 N KPF₆ solution. Following this, stirring was carried out at room temperature for 1 hour, a solid thus formed was filtered and washed twice with 50 mL of water, and recrystallized from isopropanol to give 4.5 g (yield 71%) of the Sulfonium salt A below.

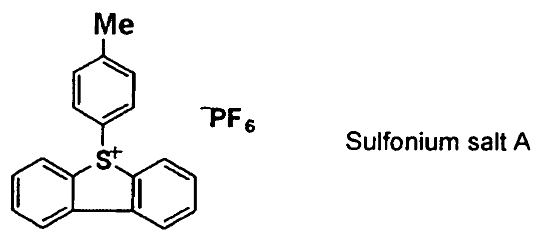

The structure of the Sulfonium salt A thus obtained was confirmed by NMR. NMR 400 MHz DMSO-d₆ 2.33 (s, 3H); 7.40 (d, 2H, J = 8.4); 7.49 (d, 2H, J = 8.4); 7.75 (dd, 2H, J = 8.0, 8.4); 7.96 (dd, 2H, J = 8.0, 8.4); 8.34 (d, 2H, J = 7.2); 8.52 (d, 2H, J = 8.4)

### Synthesis of Sulfonium salt B

### Synthesis of 2,8-dichlorodibenzothiophene oxide

150 mL of methylene chloride and 100 mL of trifluoromethanesulfonic acid were added to 7.3 g of 2,8-dichlorodibenzothiophene, and stirred at 0°C. 3.3 g of 30 wt % aqueous hydrogen peroxide was added thereto dropwise over 10 minutes, and the temperature was increased from 0°C to room temperature. After stirring at room temperature for 1 hour, the mixture was poured into 200 mL of iced water and extracted with 100 mL of methylene chloride, the aqueous phase was further extracted twice with 50 mL of methylene chloride, and this was followed by washing with saturated brine. The organic phase was concentrated, and a solid thus formed was suspended in 100 mL of methanol for 1 hour. After filtration, crystals thus obtained were vacuum-dried to give 7.1 g (yield 92%) of 2,8-dichlorodibenzothiophene oxide.

### Synthesis of Sulfonium salt B

1.5 g of 2,8-dichlorodibenzothiophene oxide was added to 10 mL of chlorobenzene and stirred. 2.2 g of aluminum chloride was added thereto and stirring was carried out at an internal temperature of 133°C for 6 hours. Following this, the mixture was poured into 50 mL of iced water and extracted with 20 mL of ethyl acetate, the aqueous phase was further extracted twice with 20 mL of ethyl acetate, and this was followed by washing with saturated brine. The organic phase was concentrated, 50 mL of acetone was added thereto, and the mixture was slowly added dropwise to 90 mL of a 0.5N KPF₆ solution. Following this, stirring was carried out at room temperature for 1 hour, and a solid thus formed was filtered. This was further washed twice with 50 mL of water and recrystallized from isopropanol to give 1.5 g (yield 53%) of the Sulfonium salt B below.

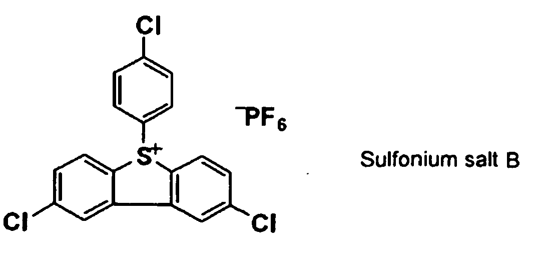

The structure of the Sulfonium salt B thus obtained was confirmed by NMR. NMR 400 MHz DMSO-d₆ 7.68 (s, 4H); 7.88 (d, 2H, J = 8.8); 8.40 (d, 2H, J = 8.8); 8.80 (s, 2H)

The ¹H-NMR chart of Sulfonium salt B is shown in FIG. 1.

### Synthesis of Sulfonium salt C

1.8 g of 2,8-dichlorodibenzothiophene oxide was added to 50 mL of toluene and stirred. The temperature was maintained at 0°C, 2.7 g of trifluoroacetic anhydride was slowly added dropwise thereto and stirred at 0°C for 30 minutes, following this 3.6 g of nonafluorobutanesulfonic acid was slowly added dropwise thereto and then stirred at 0°C for 3 hours, following this the mixture was poured into 50 mL of water and extracted with 20 mL of toluene, and the aqueous phase was further extracted with 20 mL of toluene. The organic phase was washed with saturated brine. The organic phase was concentrated, 30 mL of N-methylpyrrolidone was added thereto, and the mixture was slowly added dropwise to 90 mL of a 0.5N KPF₆ solution. Following this, stirring was carried out at room temperature for 1 hour, and a solid thus formed was filtered, washed twice with 50 mL of water, and recrystallized from acetonitrile to give 1.7 g (yield 52%) of the Sulfonium salt C below.

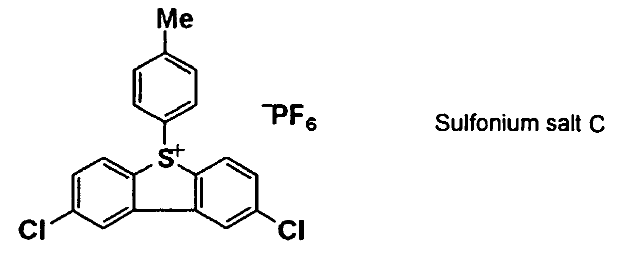

The structure of the Sulfonium salt C thus obtained was confirmed by NMR. NMR 400 MHz DMSO-d₆ 2.34 (s, 3H); 7.40 (d, 2H, J = 8.4); 7.54 (d, 2H, J = 8.4); 7.86 (d, 2H, J = 8.4); 8.36 (d, 2H, J = 8.4); 8.79 (s, 2H)

### Synthesis of Sulfonium salt D

In the synthetic method for 2,8-dichlorodibenzothiophene oxide, 2,8-dibromodibenzothiophene (manufactured by Tokyo Chemical Industry Co., Ltd.) was used instead of 2,8-dichlorodibenzothiophene, and 1.2 g (yield 65%) of Sulfonium salt D was obtained by the same method as for Sulfonium salt C.

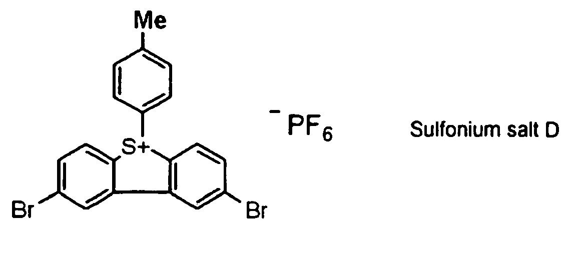

### Synthesis of Sulfonium salt E

In the synthetic method for 2,8-dichlorodibenzothiophene oxide, 2,8-dibromodibenzothiophene (manufactured by Tokyo Chemical Industry Co., Ltd.) was used instead of 2,8-dichlorodibenzothiophene, and 1.5 g (yield 60%) of Sulfonium salt E was obtained by the same method as for Sulfonium salt B.

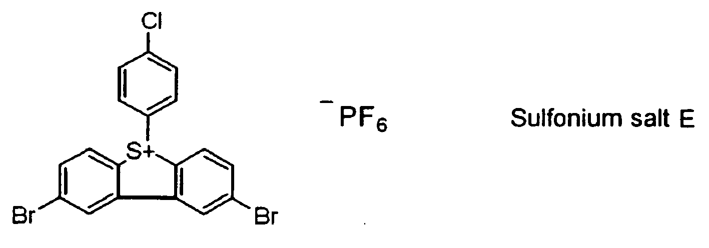

### Synthesis of Sulfonium salt F

In the synthetic method for 2,8-dichlorodibenzothiophene oxide, 2-chlorodibenzothiophene (synthesized by a method described in Bull. Chem. Soc. Jpn. 1981, 54, 2374-2378) was used instead of 2,8-dichlorodibenzothiophene, and 1.2 g (yield 40%) of Sulfonium salt F was obtained by the same method as for Sulfonium salt B.

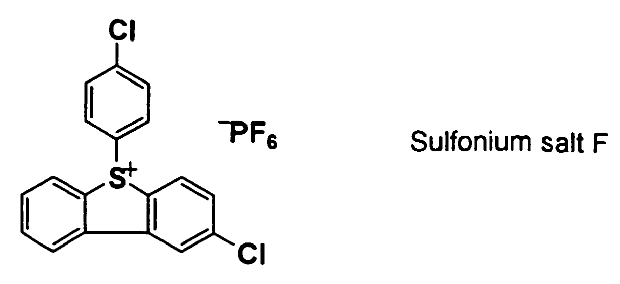

### Preparation of pigment dispersions

Yellow, magenta, cyan, black, and white pigment dispersions 1 were prepared according to the methods below. Dispersion conditions were appropriately adjusted using a known dispersing device so that the average particle size of the pigment particles in each case was in the range of 0.2 to 0.3 µm, and they were subsequently filtered using a filter while heating.

| Yellow pigment dispersion 1 | |
|---|---|
| Cl Pigment Yellow 13 | 20 parts by weight |
| Polymeric dispersant (Solsperse series, manufactured by Zeneca) | 20 parts by weight |
| OXT-221 (manufactured by Toagosei Co., Ltd.) | 60 parts by weight |

| Magenta pigment dispersion 1 | |
|---|---|
| CI Pigment Red 57:1 | 20 parts by weight |
| Polymeric dispersant (Solsperse series, manufactured by Zeneca) | 20 parts by weight |
| OXT-221 (manufactured by Toagosei Co., Ltd.) | 60 parts by weight |

| Cyan pigment dispersion 1 | |
|---|---|
| CI Pigment Blue 15:3 | 20 parts by weight |
| Polymeric dispersant (Solsperse series, manufactured by Zeneca) | 20 parts by weight |
| OXT-221 (manufactured by Toagosei Co., Ltd.) | 60 parts by weight |

| Black pigment dispersion 1 | |
|---|---|
| Cl Pigment Black 7 | 20 parts by weight |
| Polymeric dispersant (Solsperse series, manufactured by Zeneca) | 20 parts by weight |
| OXT-221 (manufactured by Toagosei Co., Ltd.) | 60 parts by weight |

| White pigment dispersion 1 | |
|---|---|
| Titanium oxide (average particle size 0.15 µm, refractive index 2.52) | 25 parts by weight |
| Neutral polymeric dispersant PB822 (manufactured by Ajinomoto-Fine-Techno Co., Inc.) | 14 parts by weight |
| OXT-221 (manufactured by Toagosei Co., Ltd.) | 60 parts by weight |

### Example 1

| Yellow ink 1 | |
|---|---|
| Yellow pigment dispersion 1 | 5 parts by weight |
| Sulfonium salt B | 6 parts by weight |
| Sensitizer: Z1 | 3 parts by weight |

| Polymerizable compounds | |
|---|---|
| Monomer: 3,4-epoxycyclohexylmethyl-3',4'-epoxycyclohexanecarboxylate (Celloxide 2021A: manufactured by Daicel-UCB Co., Ltd.) | 40 parts by weight |
| Monomer: 3,7-bis(3-oxetanyl)-5-oxanonane (OXT-221: manufactured by Toagosei Co., Ltd.) | 45 parts by weight |
| Surfactant: BYK307 (manufactured by BYK Chemie) | 1 part by weight |
| 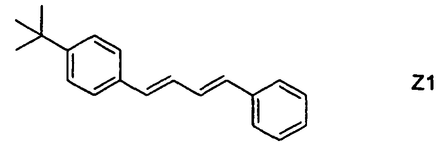 | |

| Magenta ink 1 | |
|---|---|
| Magenta pigment dispersion 1 | 5 parts by weight |
| Sulfonium salt B | 6 parts by weight |
| Sensitizer: Z1 | 3 parts by weight |

| Polymerizable compounds | |
|---|---|
| Monomer: 3,4-epoxycyclohexylmethyl-3',4'-epoxycyclohexanecarboxylate (Celloxide 2021A: manufactured by Daicel-UCB Co., Ltd.) | 40 parts by weight |
| Monomer: 3,7-bis(3-oxetanyl)-5-oxanonane (OXT-221: manufactured by Toagosei Co., Ltd.) | 45 parts by weight |
| Surfactant: BYK307 (manufactured by BYK Chemie) | 1 part by weight |

| Cyan ink 1 | |
|---|---|
| Cyan pigment dispersion 1 | 5 parts by weight |
| Sulfonium salt B | 6 parts by weight |
| Sensitizer: Z1 | 3 parts by weight |

| Polymerizable compounds | |
|---|---|
| Monomer: 3,4-epoxycyclohexylmethyl-3',4'-epoxycyclohexanecarboxylate (Celloxide 2021A: manufactured by Daicel-UCB Co., Ltd.) | 40 parts by weight |
| Monomer: 3,7-bis(3-oxetanyl)-5-oxanonane (OXT-221: manufactured by Toagosei Co., Ltd.) | 45 parts by weight |
| Surfactant: BYK307 (manufactured by BYK Chemie) | 1 part by weight |

| Black ink 1 | |
|---|---|
| Black pigment dispersion 1 | 5 parts by weight |
| Sulfonium salt B | 6 parts by weight |
| Sensitizer: Z1 | 3 parts by weight |

| Polymerizable compounds | |
|---|---|
| Monomer: 3,4-epoxycyclohexylmethyl-3',4'-epoxycyclohexanecarboxylate (Celloxide 2021A: manufactured by Daicel-UCB Co., Ltd.) | 40 parts by weight |
| Monomer: 3,7-bis(3-oxetanyl)-5-oxanonane (OXT-221: manufactured by Toagosei Co., Ltd.) | 45 parts by weight |
| Surfactant: BYK307 (manufactured by BYK Chemie) | 1 part by weight |

| White ink 1 | |
|---|---|
| White pigment dispersion 1 | 5 parts by weight |
| Sulfonium salt B | 6 parts by weight |
| Sensitizer: Z1 | 3 parts by weight |

| Polymerizable compounds | |
|---|---|
| Monomer: 3,4-epoxycyclohexylmethyl-3',4'-epoxycyclohexanecarboxylate (Celloxide 2021A: manufactured by Daicel-UCB Co., Ltd.) | 40 parts by weight |
| Monomer: 3,7-bis(3-oxetanyl)-5-oxanonane (OXT-221: manufactured by Toagosei Co., Ltd.) | 45 parts by weight |
| Surfactant: BYK307 (manufactured by BYK Chemie) | 1 part by weight |

Inks 1 of each color prepared above were filtered using a filter having an absolute filtration accuracy of 2 µm to give Inks 1 of each color.

### Inkjet image recording

Subsequently, recording was carried out on a recording medium using a commercial inkjet recording device having a piezo system inkjet nozzle. The ink supply system comprised a main tank, a supply pipe, an ink supply tank immediately before an inkjet head, a filter, and a piezo system inkjet head, and a section from the ink supply tank to the inkjet head was thermally insulated and heated. Temperature sensors were provided on the ink supply tank and in the vicinity of the nozzle of the inkjet head, and the temperature was controlled so that the nozzle section was always at 70°C ±2°C. The piezo system inkjet head was driven so as to discharge multisize dots of 8 to 30 pL at a resolution of 720 x 720 dpi. The exposure system, the main scanning speed, and the discharge frequency were adjusted so that, after landing, UV light was focused to give an exposure area illumination intensity of 100 mW/cm², and irradiation started 0.1 sec. after the ink landed on the recording medium. Furthermore, the exposure time was made variable, and exposure energy was applied. Here, dpi referred to in the present invention denotes the number of dots per 2.54 cm.

The inks of each color prepared above were discharged at an environmental temperature of 25°C in the order black → cyan → magenta → yellow → white, and irradiation with ultraviolet rays was carried out using a VZero 085 metal halide lamp manufactured by Integration Technology after each color was discharged. As an energy level that could completely cure the inks so that tackiness disappeared when touched by hand, the total exposure energy per color was 100 mJ/cm² for all the colors. As recording media, a grained aluminum support, a transparent biaxially stretched polypropylene film whose surface had been treated so as to impart printability, a soft vinyl chloride sheet, a cast coat paper, and a commercial recycled paper were used, each color image was recorded, and an image having high resolution without dot spreading was obtained in all cases. Furthermore, for high quality paper, the ink did not penetrate to the reverse side, the ink was sufficiently cured, and there was hardly any odor due to unreacted monomer. Moreover, the ink recorded on the film had sufficient flexibility, the ink did not crack when bent, and there was no problem in an adhesion test involving peeling with Sellotape (trademark).

### Examples 2 to 12 and Comparative Examples 1 to 8

### Preparation of ink

White inks 2 to 16 were prepared in accordance with the methods described below.

### Example 2: White ink 2

White ink 2 was prepared in the same manner as for White ink 1 except that Sulfonium salt C was used instead of Sulfonium salt B.

### Example 3: White ink 3

White ink 3 was prepared in the same manner as for White ink 1 except that Sulfonium salt D was used instead of Sulfonium salt B.

### Example 4: White ink 4

White ink 4 was prepared in the same manner as for White ink 1 except that Sulfonium salt E was used instead of Sulfonium salt B.

### Example 5: White ink 5

White ink 5 was prepared in the same manner as for White ink 1 except that Sulfonium salt F was used instead of Sulfonium salt B.

### Example 6: White ink 6

White ink 6 was prepared in the same manner as for White ink 1 except that 3 parts by weight of octylamine was added as a basic compound to White ink 1.

### Example 7: White ink 7

White ink 7 was prepared in the same manner as for White ink 1 except that Z1 of White ink 1 was replaced with 9,10-dibutoxyanthracene.

### Example 8: White ink 8

| | |
|---|---|
| White pigment dispersion 1 | 5 parts by weight |
| Sulfonium salt B | 6 parts by weight |
| Sensitizer: Z1 | 3 parts by weight |

| Polymerizable compounds | |
|---|---|
| Monomer: 3,4-epoxycyclohexylmethyl-3',4'-epoxycyclohexanecarboxylate (Celloxide 2021A: manufactured by Daicel-UCB Co., Ltd.) | 30 parts by weight |
| Monomer: 3,7-bis(3-oxetanyl)-5-oxanonane (OXT-221: manufactured by Toagosei Co., Ltd.) | 45 parts by weight |
| Surfactant BYK307 (manufactured by BYK Chemie) | 1 part by weight |
| Radically polymerizable monomer: 1,6-hexanedioldiacrylate | 10 parts by weight |

### Comparative Example 1: White ink 9

| | |
|---|---|
| White pigment dispersion 1 | 5 parts by weight |
| UVI-6992 (manufactured by The Dow Chemical Company) | 6 parts by weight |
| Sensitizer: Z1 | 3 parts by weight |

| Polymerizable compounds | |
|---|---|
| Monomer: 3,4-epoxycyclohexylmethyl-3',4'-epoxycyclohexanecarboxylate(Celloxide 2021A: manufactured by Daicel-UCB Co., Ltd.) | 40 parts by weight |
| Monomer: 3,7-bis(3-oxetanyl)-5-oxanonane (OXT-221: manufactured by Toagosei Co., Ltd.) | 45 parts by weight |
| Surfactant: BYK307 (manufactured by BYK Chemie) | 1 part by weight |

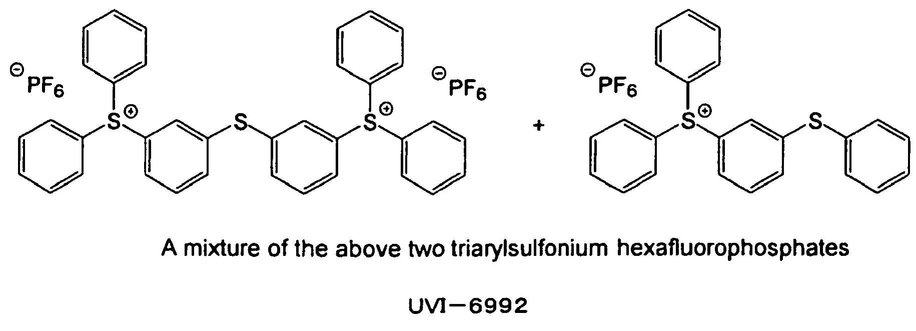

### Comparative Example 2: White ink 10

| | |
|---|---|
| White pigment dispersion 1 | 5 parts by weight |
| IRGACURE 250 (manufactured by Ciba Specialty Chemicals) 6 parts by weight Sensitizer: Z1 | 3 parts by weight |

| Polymerizable compounds | |
|---|---|
| Monomer: 3,4-epoxycyclohexylmethyl-3',4'-epoxycyclohexanecarboxylate (Celloxide 2021A: manufactured by Daicel-UCB Co., Ltd.) | 40 parts by weight |
| Monomer: 3,7-bis(3-oxetanyl)-5-oxanonane (OXT-221: manufactured by Toagosei Co., Ltd.) | 45 parts by weight |
| Surfactant: BYK307 (manufactured by BYK Chemie) | 1 part by weight |

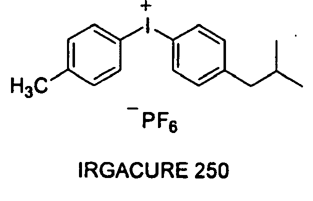

### Comparative Example 3: White ink 11

White ink 11 was prepared in the same manner as for White ink 9 except that Sulfonium salt A was used instead of the initiator UVI-6992 (manufactured by The Dow Chemical Company) of White ink 9.

### Comparative Example 4: White ink 12

White ink 12 was prepared in the same manner as for White ink 9 except that Esacure 1187 (Lamberti) was used instead of the initiator UVI-6992 (manufactured by The Dow Chemical Company) of White ink 9.

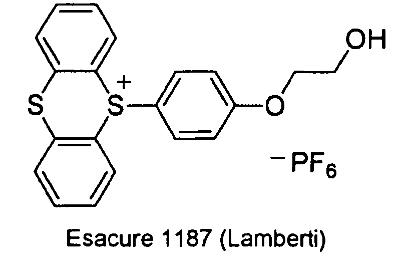

### Example 9: White ink 13

| Preparation of White pigment dispersion 2 | |
|---|---|
| Titanium oxide (average particle size 0.15 µm, refractive index 2.52) | 25 parts by weight |
| Neutral polymeric dispersant PB822 (manufactured by Ajinomoto-Fine-Techno Co., Inc.) | 14 parts by weight |
| Diethylene glycol.divinyl ether (manufactured by BASF) | 60 parts by weight |

| White ink 13 | |
|---|---|
| White pigment dispersion 2 | 5 parts by weight |
| Sulfonium salt B | 6 parts by weight |
| Sensitizer: Z1 | 3 parts by weight |

| Radically polymerizable compounds | |
|---|---|
| Caprolactone-modified dipentaerythritol hexaacrylate (DPCA60, manufactured by SARTOMER; polymerizable compound) | 30 parts by weight |
| 1,6-Hexanediol diacrylate (HDDA, manufactured by Daicel-UCB Co., Ltd.; polymerizable compound) | 55 parts by weight |
| Polymerization inhibitor (Al cupferron, manufactured by Wako Pure Chemical Industries, Ltd.) | 1 part by weight |

### Comparative Example 5: White ink 14

White ink 14 was prepared in the same manner as for White ink 13 except that the sulfonium salt UVI-6992 (manufactured by The Dow Chemical Company) was used instead of Sulfonium salt B of White ink 13.

### Comparative Example 6: White ink 15

White ink 15 was prepared in the same manner as for White ink 14 except that Sulfonium salt A was used instead of the initiator UVI-6992 (manufactured by The Dow Chemical Company) of White ink 14.

### Comparative Example 7: White ink 16

White ink 16 was prepared in the same manner as for White ink 14 except that Esacure 1187 (Lamberti) was used instead of the initiator UVI-6992 (manufactured by The Dow Chemical Company) of White ink 14.

Crude white inks 2 to 16 prepared as described above were filtered using a filter having an absolute filtration accuracy of 2 µm to give White inks 2 to 16.

### Example 10: Magenta ink 2

Magenta ink 2 was prepared in the same manner as for Magenta ink 1 except that instead of Cl pigment red 57:1 Compound M-1 below (oxidation potential +1.37 V) was used as an oil-soluble dye, and filtration was carried out using a filter having an absolute filtration accuracy of 2 µm.

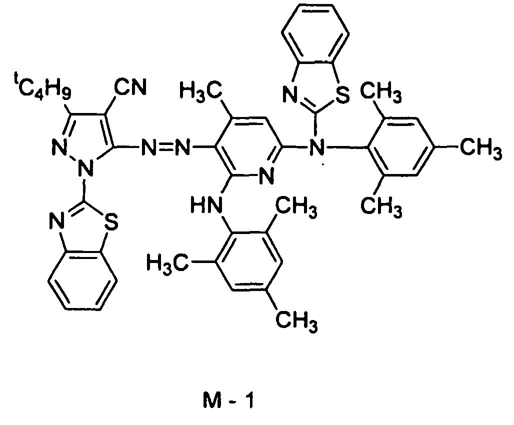

### Example 11: Magenta ink 3

Magenta ink 3 was prepared in the same manner as for Magenta ink 1 except that instead of Cl pigment red 57:1 Compound M-2 below (oxidation potential +0.94 V) was used as an oil-soluble dye, and filtration was carried out using a filter having an absolute filtration accuracy of 2 µm.

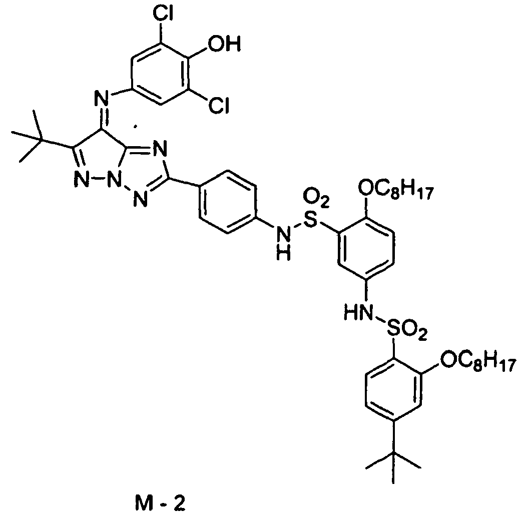

### Inkjet image recording

White and magenta images were formed by the same method as described in Example 1 using the White inks 1 to 16 and Magenta inks 1 to 3 prepared above.

### Example 12

A white image was formed in the same manner as in Example 1 using White ink 1 prepared in Example 1 except that a UV light-emitting diode (UV-LED) was used instead of the VZero 085 metal halide lamp manufactured by Integration Technology.

In this embodiment, an NCCU033 manufactured by Nichia Corporation was used as the UV-LED. The LED emits UV light at a wavelength of 365 nm from 1 chip, and by applying a current of about 500 mA, light of about 100 mW is emitted from the chip. A plurality thereof were aligned at intervals of 7 mm to give a power of 0.3 W/cm² on the surface of a recording medium (hereinafter, also called a medium). The time from landing to irradiation and the exposure time can be varied by the transport speed of the medium and the distance between a head and the LED in the transport direction. In this embodiment, irradiation was carried out about 0.5 sec. after landing.

The exposure energy on the medium was adjustable in the range of 0.01 to 15 J/cm² by setting the distance from the medium and the transport speed.

### Comparative Example 8

A white image was formed in the same manner as in Example 12 using White ink 9.

### Inkjet image evaluation

Subsequently, with regard to each of the images thus formed, the sensitivity required for curing, storage stability, and plate life were evaluated in accordance with the methods described below.

### 1. Curing sensitivity measurement

High quality paper was superimposed on a printed sample immediately after exposure, they were passed through pressure rollers (50 kg/cm²), transfer of coloring material onto the high quality paper was evaluated, and the exposure energy intensity (mJ/cm²) when transfer did not occur was defined as the curing sensitivity. The smaller the value, the higher the sensitivity.

When measuring curing sensitivity, a grained aluminum support was used as a recording medium.

### 2. Storage stability evaluation

After storing a prepared ink at 75%RH and 60°C for 3 days, the ink viscosity at the discharge temperature was measured, and an increase in the ink viscosity was expressed as a viscosity ratio (after storage/before storage). When the viscosity was unchanged and the ratio was close to 1.0, the storage stability was good, and if the ratio exceeded 1.5, clogging might undesirably be caused during discharge.

### 3. Evaluation of plate life

An image printed on a grained aluminum support prepared above was used as a printing plate, printing was carried out using a Heidel KOR-D machine, and a relative comparison of the number of prints completed was used as an index for the plate life (the number obtained for Example 1 was defined as 100). The larger the number, the longer the plate life, which is preferable.

### 4. Evaluation of discharge stability

An ink was continuously discharged using the above-mentioned printer, and discharge stability was evaluated in accordance with the criteria below. In the evaluation ranking below, when it was evaluated as Good, it was judged that there would be no problem in actual application.
Good: no missing nozzles occurred even after 1 hour or longer of continuous discharge.
Fair: no missing nozzles occurred after at least 30 minutes but less than 1 hour of continuous discharge.
Poor: missing nozzles occurred after less than 30 minutes of continuous discharge.

### 5. Evaluation of coloration

After a white ink was printed on a PET substrate, it was superimposed on a white board, samples were visually evaluated by 10 panelists with respect to 3 levels, and an average score was calculated.
3 points: no yellowish color.
2 points: slight yellowish color.
1 point: strong yellowish color.

**(Table 2)**

| Example | Ink No. | Sulfonium salt | Curing (mJ/cm²) | Storage stability | Plate life | Discharge stability | Coloration |
|---|---|---|---|---|---|---|---|
| Ex. 1 | Magenta 1 | B | 80 | 1.1 | 100 | Good | - |
| Ex. 1 | White 1 | B | 80 | 1.1 | 100 | Good | 3 |
| Ex. 2 | White 2 | C | 80 | 1.1 | 100 | Good | 3 |
| Ex. 3 | White 3 | D | 80 | 1.1 | 100 | Good | 3 |
| Ex. 4 | White 4 | E | 80 | 1.1 | 100 | Good | 3 |
| Ex. 5 | White 5 | F | 90 | 1.1 | 90 | Good | 3 |
| Ex. 6 | White 6 | B | 85 | 1 | 95 | Good | 3 |
| Ex. 7 | White 7 | B | 90 | 1.1 | 95 | Good | 2 |
| Ex. 8 | White 8 | B | 80 | 1.1 | 120 | Good | 3 |
| Ex. 9 | White 13 | B | 150 | 1 | 85 | Good | 3 |
| Ex. 10 | Magenta 2 | B | 80 | 1.1 | 150 | Good | - |
| Ex. 11 | Magenta 3 | B | 70 | 1 | 160 | Good | - |
| Ex. 12 | White 1 | B | 75 | 1.1 | 120 | Good | 3 |
| Comp. Ex. 1 | White 9 | UVI-6992 | 200 | 1.1 | 70 | Poor | 2 |
| Comp. Ex. 2 | White 10 | IRGACURE 250 | 70 | 1.5 | 160 | Poor | 2 |
| Comp. Ex. 3 | White 11 | A | 100 | 1.1 | 80 | Poor | 3 |
| Comp. Ex. 4 | White 12 | Esacure 1187 | 110 | 1.1 | 80 | Poor | 2 |
| Comp. Ex. 5 | White 14 | UVI-6992 | 300 | 1.1 | 50 | Poor | 2 |
| Comp. Ex. 6 | White 15 | A | 250 | 1.1 | 55 | Poor | 3 |
| Comp. Ex. 7 | White 16 | Esacure 1187 | 270 | 1.1 | 55 | Poor | 2 |
| Comp. Ex. 8 | White 9 | UVI-6992 | 300 | 1.1 | 50 | Poor | 2 |

### Curable composition

An epoxy group-containing polycarboxylic acid resin was synthesized as follows.

| | |
|---|---|
| Phenol novolac type epoxy resin (EPPN-503, epoxy equivalent weight 180.5, manufactured by Nippon Kayaku Co., Ltd.) | 361.0 g (2 equivalent weight) |
| Dimethylolpropionic acid | 134.1 g (1 mol) |
| Carbitol acetate | 86.7 g |
| Solvent naphtha | 37.1 g |

The above-mentioned components were weighed and heated at 90°C while stirring to thus dissolve a reaction mixture. The reaction mixture was then cooled to 60°C, 1.86 g (0.007 mol) of triphenylphosphine was added thereto, the mixture was heated to 100°C and reacted for about 20 hours, when the acid value became 0.7 mg KOH/g or less it was cooled to 50°C, 1.07 g (0.007 mol) of cumene hydroperoxide, 169.4 g of carbitol acetate, and 72.6 g of solvent naphtha were added, the mixture was reacted for about 3 hours, and triphenylphosphine, which is a reaction catalyst, was oxidized so as to deactivate its catalytic activity.

Following this, 184.2 g (1.21 mol) of tetrahydrophthalic anhydride was added, and the mixture was reacted at 95°C for 10 hours to give an epoxy group-containing polycarboxylic acid resin (solids concentration 65%). The acid value of the product (solids content) was about 100 mg KOH/g.

The epoxy group-containing polycarboxylic acid resin thus obtained was used to prepare the resin compositions shown in Table 3 below.

The resin composition was spin-coated on a glass substrate at 1,000 rpm for 30 seconds, pre-baked in an oven at 80°C for 30 minutes, and printed with a step tablet (No. 2, manufactured by Kodak) using a high pressure mercury lamp at an exposure of 2,000 mJ/cm². Following this, it was post-baked in an oven at 90°C for 10 minutes and then developed using a 1% aqueous solution of sodium carbonate, and the step number at which the cured film remained without being dissolved by development, that is, there was residual film, was ascertained. From the results, residual film was only observed up to step 5 (exposure 316 mJ/cm²) and step 6 (exposure 223 mJ/cm²) for Comparative Examples 9 and 10, whereas in Example 13, in which Compound B was used, residual film was observed up to step 9 (exposure 79 mJ/cm²), and it was confirmed that Compound B clearly had an effect in increasing the sensitivity.

**(Table 3)**

| | Ex. 13 | Comp. Ex. 9 | Comp. Ex. 10 |
|---|---|---|---|
| Epoxy group-containing polycarboxylic acid resin | 5 g | 5 g | 5 g |
| Propylene glycol monomethyl ether acetate | 3.86 g | 3.86 g | 3.86 g |
| Initiator | Compound B | Esacure 1064 | Esacure 1187 |
| | | (Lamberti) | (Lamberti) |
| | 0.25 g | 0.49 g^{*1} | 0.38 g^{*2} |
| 9,10-Dibutoxyanthracene | 0.25 g | 0.25 g | 0.25 g |
| Sensitivity | Step 9 | Step 5 | Step 6 |
| | Exposure | Exposure | Exposure |
| | 79 mJ/cm² | 316 mJ/cm² | 223 mJ/cm² |

| | | | |
|---|---|---|---|
| *1 Effective component 50 wt % *2 Effective component 75 wt % | | | |

## Claims

1. A sulfonium salt having a cation represented by Formula (II), 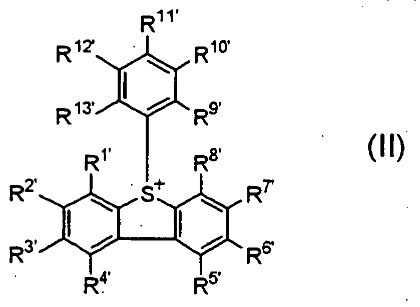 (R^{1'}to R^{13'} in Formula (II) independently denote a hydrogen atom or a substituent, and may be bonded to each other to form a ring, provided that at least one of R^{1'} to R^{8'} denotes a halogen atom or a haloalkyl group).

2. The sulfonium salt according to Claim 1, wherein R^{1'} to R^{13'} in Formula (II) independently denote one selected from the group consisting of a hydrogen atom, a halogen atom, an alkyl group, an alkenyl group, an alkynyl group, an aryl group, a cyano group, a hydroxyl group, a carboxyl group, an alkoxy group, an aryloxy group, an acyloxy group, a carbamoyloxy group, an acylamino group, an aminocarbonylamino group, an alkoxycarbonylamino group, an aryloxycarbonylamino group, a sulfamoylamino group, an alkyl or aryl sulfonylamino group, an alkylthio group, an arylthio group, a sulfamoyl group, an alkyl or aryl sulfonyl group, an aryloxycarbonyl group, an alkoxycarbonyl group, a carbamoyl group, an imide group, a silyl group and a ureido group.

3. The sulfonium salt according to Claim 1 or 2, wherein R^{1'} to R^{8'} in Formula (II) independently denote one selected from the group consisting of a hydrogen atom, a halogen atom and a haloalkyl group; and R^{9'} to R^{13'} in Formula (II) independently denote one selected from the group consisting of a hydrogen atom, an alkyl group, a cyano group, a hydroxyl group, an alkoxy group, an alkylsulfonyl group and an alkylthio group.

4. The sulfonium salt according to any one of Claims 1 to 3, wherein at least two of R^{1'} to R^{8'} in Formula (II) denote a halogen atom or a haloalkyl group.

5. A curable composition comprising the sulfonium salt according to any one of Claims 1 to 4.

6. A curable composition comprising:
(a) the sulfonium salt according to any one of Claims 1 to 4;
(b) a polymerizable compound; and
(c) a sensitizer.

7. An ink composition comprising the curable composition according to Claim 5 or 6.

8. The ink composition according to Claim 7, wherein it comprises (d) a colorant.

9. An inkjet recording method comprising:
a step (a¹) of discharging an ink composition onto a recording medium; and
a step (b¹) of irradiating the discharged ink composition with actinic radiation so as to cure the ink composition,
the ink composition being the ink composition according to Claim 7 or 8.

10. A printed material recorded by the inkjet recording method according to Claim 9.

11. A process for producing a lithographic printing plate, the process comprising:
a step (a²) of discharging the ink composition according to Claim 7 or 8 onto a hydrophilic support; and
a step (b²) of irradiating the discharged ink composition with actinic radiation so as to cure the ink composition, thus forming a hydrophobic image on the hydrophilic support by curing the ink composition.

12. A lithographic printing plate produced by the process for producing a lithographic printing plate according to Claim 11.

## Patentansprüche

1. Sulfoniumsalz mit einem Kation der Formel (II) 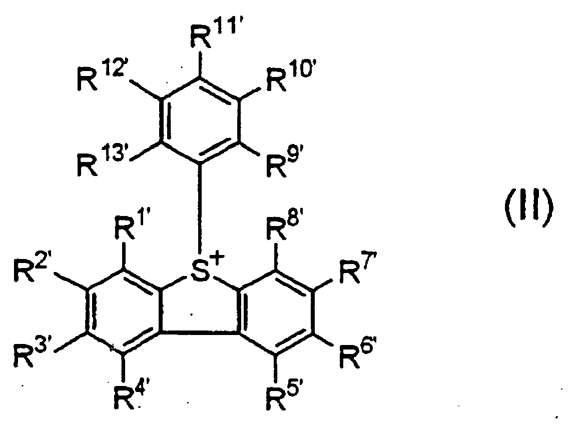 (R^{1'} bis R^{13'} in Formel (II) bezeichnen unabhängig ein Wasserstoffatom oder einen Substituenten, und können aneinander unter Bildung eines Rings gebunden sein, vorausgesetzt, dass mindestens eines von R^{1'} bis R^{8'} ein Halogenatom oder eine Haloalkylgruppe bezeichnet).

2. Sulfoniumsalz nach Anspruch 1, worin R^{1'} bis R^{13'} in Formel (II) unabhängig eines ausgewählt aus der Gruppe bezeichnen, die aus einem Wasserstoffatom, einem Halogenatom, einer Alkylgruppe, einer Alkenylgruppe, einer Alkinylgruppe, einer Arylgruppe, einer Cyanogruppe, einer Hydroxylgruppe, einer Carboxylgruppe, einer Alkoxygruppe, einer Aryloxygruppe, einer Acyloxygruppe, einer Carbamoyloxygruppe, einer Acylaminogruppe, einer Aminocarbonylaminogruppe, einer Alkoxycarbonylaminogruppe, einer Aryloxycarbonylaminogruppe, einer Sulfamoylaminogruppe, einer Alkyl- oder Arylsulfonylaminogruppe, einer Alkylthiogruppe, einer Arylthiogruppe, einer Sulfamoylgruppe, einer Alkyl- oder Arylsulfonylgruppe, einer Aryloxycarbonylgruppe, einer Alkoxycarbonylgruppe, einer Carbamoylgruppe, einer Imidgruppe, einer Silylgruppe und einer Ureidogruppe besteht.

3. Sulfoniumsalz nach Anspruch 1 oder 2, worin R^{1'} bis R^{8'} in Formel (II) unabhängig eines bezeichnen, das aus der Gruppe ausgewählt ist, die aus einem Wasserstoffatom, einem Halogenatom und einer Haloalkylgruppe besteht; und R^{9'} bis R^{13'} in Formel (II) unabhängig eines bezeichnen, das aus der Gruppe ausgewählt ist, die aus einem Wasserstoffatom, einer Alkylgruppe, einer Cyanogruppe, einer Hydroxylgruppe, einer Alkoxygruppe, einer Alkylsulfonylgruppe und einer Alkylthiogruppe besteht.

4. Sulfoniumsalz nach einem der Ansprüche 1 bis 3, worin mindestens zwei aus R^{1'} bis R^{8'} in Formel (II) ein Halogenatom oder eine Haloalkylgruppe bezeichnen.

5. Härtbare Zusammensetzung, die das Sulfoniumsalz nach einem der Ansprüche 1 bis 4 umfasst.

6. Härtbare Zusammensetzung, die umfasst:
(a) das Sulfoniumsalz nach einem der Ansprüche 1 bis 4,
(b) eine polymerisierbare Verbindung; und
(c) ein Sensibilisierungsmittel.

7. Tintenzusammensetzung, die die härtbare Zusammensetzung nach Anspruch 5 oder 6 umfasst.

8. Tintenzusammensetzung nach Anspruch 7, worin diese ein Färbemittel (d) umfasst.

9. Tintenstrahlaufzeichnungsverfahren umfassend:
einen Schritt (a¹) zum Entladen einer Tintenzusammensetzung auf ein Aufzeichnungsmedium; und
einen Schritt (b¹) zum Bestrahlen der entladenen Tintenzusammensetzung mit aktinischer Bestrahlung, um so die Tintenzusammensetzung zu härten,
wobei die Tintenzusammensetzung die Tintenzusammensetzung nach Anspruch 7 oder 8 ist.

10. Gedrucktes Material, das durch das Tintenstrahlaufzeichnungsverfahren nach Anspruch 9 aufgezeichnet wurde.

11. Verfahren zur Herstellung einer lithographischen Druckplatte, wobei das Verfahren umfasst:
einen Schritt (a²) zum Entladen der Tintenzusammensetzung nach Anspruch 7 oder 8 auf einen hydrophilen Träger; und
einen Schritt (b²) zum Bestrahlen der entladenen Tintenzusammensetzung mit aktinischer Bestrahlung, um so die Tintenzusammensetzung zu härten, wobei so ein hydrophobes Bild auf dem hydrophilen Träger durch Härten der Tintenzusammensetzung gebildet wird.

12. Lithographische Druckplatte, die durch das Verfahren zur Herstellung einer lithographischen Druckplatte nach Anspruch 11 hergestellt wurde.

## Revendications

1. Sel de sulfonium ayant un cation représenté par la Formule (II), 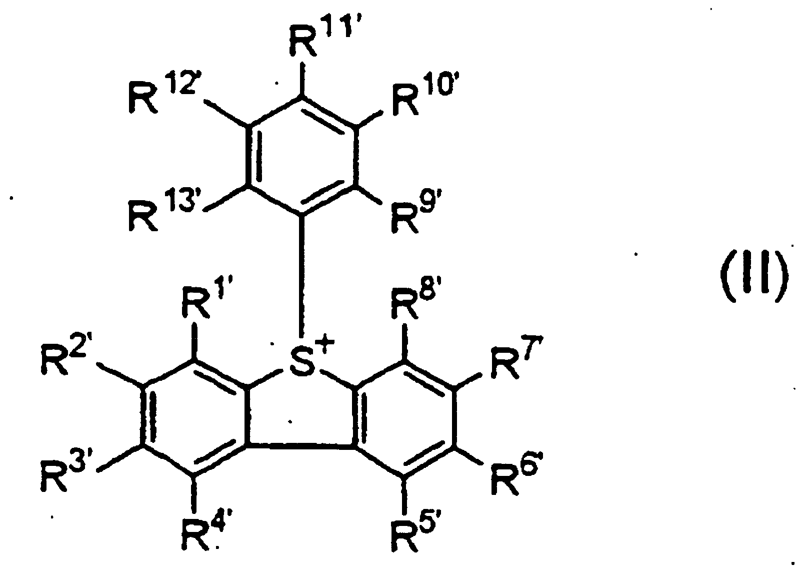 (R^{1'} à R^{13'} dans la Formule (II) représentent indépendemment un atome d'hydrogène ou un substituant, et peuvent être liés l'un à l'autre pour former un cycle, à la condition qu'au moins l'un parmi R^{1'} à R^{8'} représente un atome d'halogène ou un groupe haloalkyle).

2. Sel de sulfonium selon la revendication 1, dans lequel R^{1'} à R^{13'} dans la Formule (II) représentent indépendemment l'un sélectionné dans le groupe constitué par un atome d'hydrogène, un atome d'halogène, un groupe alkyle, un groupe alcényle, un groupe alcynyle, un groupe aryle, un groupe cyano, un groupe hydroxyle, un groupe carboxyle, un groupe alcoxy, un groupe aryloxy, un groupe acyloxy, un groupe carbamoyloxy, un groupe acylamino, un groupe aminocarbonylamino, un groupe alcoxycarbonylamino, un groupe aryloxycarbonylamino, un groupe sulfamoylamino, un groupe alkyl- ou arylsulfonylamino, un groupe alkylthio, un groupe arylthio, un groupe sulfamoyle, un groupe alkyl- ou aryl-sulfonyle, un groupe aryloxycarbonyle, un groupe alcoxycarbonyle, un groupe carbamoyle, un groupe imide, un groupe silyle et un groupe uréido.

3. Sel de sulfonium selon la revendication 1 ou 2, dans lequel R^{1'} à R^{8'} dans la Formule (II) représentent indépendemment l'un sélectionné dans le groupe constitué par un atome d'hydrogène, un atome d'halogène, et un groupe haloalkyle ; et R^{9'} à R^{13'} dans la Formule (II) représentent indépendemment l'un sélectionné dans le groupe constitué par un atome d'hydrogène, un groupe alkyle, un groupe cyano, un groupe hydroxyle, un groupe alcoxy, un groupe alkylsulfonyle et un groupe alkylthio.

4. Sel de sulfonium selon l'une quelconque des revendications 1 à 3, dans lequel au moins deux de R^{1'} à R^{8'} dans la Formule (II) représentent un atome d'halogène ou un groupe haloalkyle.

5. Composition durcissable comprenant le sel de sulfonium selon l'une quelconque des revendications 1 à 4.

6. Composition durcissable comprenant :
(a) le sel de sulfonium selon l'une quelconques des revendications 1 à 4 ;
(b) un composé polymérisable ; et
(c) un sensibilisateur.

7. Composition d'encre comprenant la composition durcissable selon la revendication 5 ou 6.

8. Composition d'encre selon la revendication 7, ladite composition comprenant (d) un colorant.

9. Procédé d'enregistrement par jet d'encre comprenant:
une étape (a¹) de libération d'une composition d'entre sur un milieu d'enregistrement ; et
une étape (b¹) d'irradiation de la composition d'encre libérée par un rayonnement actinique de manière à durcir la composition d'encre,
la composition d'encre étant la composition d'encre selon la revendication 7 ou 8.

10. Matériau imprimé enregistré par le procédé d'enregistrement par jet d'encre selon la revendication 9.

11. Procédé pour produire une plaque d'impression lithographique, le procédé comprenant :
une étape (a²) de libération de la composition d'encre selon la revendication 7 ou 8 sur un support hydrophile ; et
une étape (b²) d'irradiation de la composition d'encre libérée par un rayonnement actinique de manière à durcir la composition d'encre, en formant ainsi une image hydrophobe sur le support hydrophile par durcissement de la composition d'encre.

12. Plaque d'impression lithographique produite par le procédé pour produire une plaque d'impression lithographique selon la revendication 11.
